(19) Europäisches Patentamt
European Patent Office
Office européen des brevets

(11) Publication number: **0 647 632 A1**

# (12) EUROPEAN PATENT APPLICATION published in accordance with Art. 158(3) EPC

(21) Application number: **93913562.0**

(22) Date of filing: **22.06.93**

(86) International application number: **PCT/JP93/00844**

(87) International publication number: **WO 94/00438 (06.01.94 94/02)**

(51) Int. Cl.[6]: **C07D 243/24**, A61K 31/55

(30) Priority: **24.06.92 JP 189826/92**

(43) Date of publication of application: **12.04.95 Bulletin 95/15**

(84) Designated Contracting States: **AT BE CH DE DK ES FR GB GR IE IT LI LU NL PT SE**

(71) Applicant: **YAMANOUCHI PHARMACEUTICAL CO. LTD.**
**No. 3-11 Nihonbashi-Honcho, 2-chome**
**Chuo-ku**
**Tokyo 103 (JP)**
Applicant: **FERRING B.V.**
**Hulswitweg 6**
**NL-2003 NL Haarlem (NL)**

(72) Inventor: **SATOH, Masato**
**5-9-321, Ninomiya 2-chome**
**Tsukuba-shi,**
**Ibaraki 305 (JP)**
Inventor: **OKAMOTO, Yoshinori**
**5-9-207, Ninomiya 2-chome, Tsukuba-shi**
**Ibaraki 305 (JP)**
Inventor: **KOSHIO, Hiroyuki**
**5-9-228, Ninomiya 2-chome**
**Tsukuba-shi,**
**Ibaraki 305 (JP)**
Inventor: **NISHIDA, Akito**
**1029-37, Oaza Kakioka,**
**Yasato-cho**
**Niihari-gun,**
**Ibaraki 315-01 (JP)**
Inventor: **MIYATA, Keiji**
**15-5-101, Azuma 4-chome**
**Tsukuba-shi,**
**Ibaraki 305 (JP)**
Inventor: **OHTA, Mitsuaki**
**9-11, Kinunodai 3-chome,**
**Yawaramura**
**Tsukuba-gun,**
**Ibaraki 300-24 (JP)**
Inventor: **RYDER, Hamish**
**79 Dean Road**
**Bitterne,**
**Southampton SO2 5AO (GB)**
Inventor: **KENDRICK, David, A.**
**258 Southampton Road**
**Eastleigh,**
**Hampshire SO5 5OU (GB)**
Inventor: **SEMPLE, Graeme**
**3 Station Road,**
**Romsey,**
**Hampshire, S051.8DP, (GB)**
Inventor: **SZELKE, Michael**
**"Southview"**
**Braishfield,**
**Romsey SO51 0PN (GB)**

(74) Representative: **Geering, Keith Edwin et al**
**REDDIE & GROSE**
**16 Theobalds Road**
**London WC1X 8PL (GB)**

## (54) NOVEL BENZODIAZEPINE DERIVATIVE.

EP 0 647 632 A1

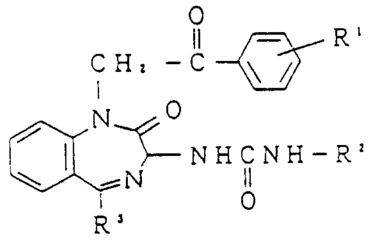

(I)

(57) A novel benzodiazepine derivative represented by general formula (I) or a pharmaceutically acceptable salt thereof. These compounds are useful as medicines, in particular, for treating and preventing diseases in which CCK-B receptor and gastrin receptor participate, because they have CCK-B receptor antagonism, gastrin receptor antagonism and the effect of inhibiting gastric juice secretion.

## TECHNICAL FIELD

This invention relates to a pharmaceutical agent, in particular, a novel benzodiazepine derivative or a salt thereof with a pharmaceutically acceptable acid or base having a CCK-B receptor antagonistic activity and/or a gastrin receptor antagonistic activity.

## TECHNICAL BACKGROUND

With respect to benzodiazepine derivatives, various synthetic studies have been made for the purpose of developing drugs having an activity on the central nervous system (psychotropic agents) which act on a benzodiazepine receptor. Recently, some of the benzodiazepine derivatives have been reported to exhibit a CCK-A (cholecystokinin-A) receptor antagonistic activity and a CCK-B (cholecystokinin-B) receptor antagonistic activity.

Also, it is reported that a compound having a high CCK-B receptor antagonistic activity inhibits the gastric acid secretion induced by pentagastrin (*Eur. J. Pharmacol.,* 162, 273-280, 1989).

## DISCLOSURE OF THE INVENTION

The present inventors disclosed, in an earlier application (WO92/11246), novel benzodiazepine derivatives which are apparently different in the chemical structure from the conventionally known compounds described in the unexamined published Japanese Patent Application No. Sho-63-238069 and which have markedly excellent effects in the pharmacological activity. The above-described compounds have markedly excellent pharmacological effects and hence are expected to have clinical effects. However, similar to the conventionally known benzodiazepine derivatives, these compounds have a problem with absorption by oral administration.

In view of the above problem, the present inventors made extensive studies on the synthesis of analogues, and found novel benzodiazepine derivatives which are expected to have excellent activities even by oral administration and completed the present invention.

That is, the present invention relates to novel benzodiazepine derivatives represented by the general formula (I) or salts thereof:

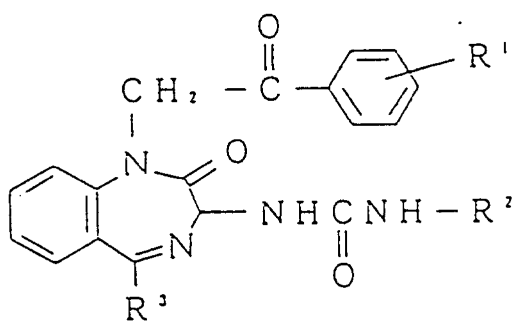

(I)

wherein $R^1$ represents a hydrogen atom, a lower alkyl group or a hydroxyl group,

$R^2$ represents (a) a phenyl group substituted with at least one of the following substituents: an amino group which may be substituted, a lower alkyl group, a cyano group, a nitro group, a carboxyl group, a lower alkoxycarbonyl group, a carbamoyl group which may be substituted or a 3- to 7-membered nitrogen-containing heterocyclic group; (b) a pyridyl group; or (c) a benzimidazolyl group, and

$R^3$ represents a phenyl group or a pyridyl group,

with the proviso that, when $R^1$ is a hydrogen atom or a lower alkyl group and $R^2$ is a lower alkyl-substituted phenyl group, then $R^3$ is a pyridyl group.

The compounds of the present invention are further described hereinafter. The lower alkyl group means a straight chain or branched chain saturated hydrocarbon group having from 1 to 6 carbon atoms, and includes, for example, a methyl group, an ethyl group, a propyl group, an isopropyl group, a butyl group, an isobutyl group, a pentyl group, a tert-pentyl group and a hexyl group.

The phenyl group substituted at least one substituent has, for example, the following groups as the substituent.

The amino group which may be substituted includes an unsubstituted amino group and an amino group substituted with one or two lower alkyl groups and/or lower acyl groups.

Of the above-described amino groups which may be substituted, a mono- or di-lower alkylamino group means an amino group having one or two alkyl groups each having from 1 to 6 carbon atoms and includes, for example, a methylamino group, an ethylamino group, a propylamino group, an isopropylamino group, a butylamino group, an isobutylamino group, a tert-butylamino group, a pentylamino group, an isopentylamino group, a tert-pentylamino group, a hexylamino group, a dimethylamino group, a diethylamino group, and an ethylmethylamino group.

Also, a mono- or di-lower-acylamino group means an amino group having one or two acyl groups each having from 1 to 6 carbon atoms and includes, for example, a formylamino group, an acetylamino group, a propionylamino group, a butyrylamino group, an isobutyrylamino group, a valerylamino group, an isovalerylamino group, a pivaloylamino group, a pentanoylamino group, diformylamino group, a diacetylamino group and a dipropionylamino group.

A lower acyl lower alkylamino group includes, for example, a formylmethylamino group, an acetylmethylamino group, a propionylmethylamino group, a formylethylamino group, an acetylethylamino group and a propionylethylamino group.

A lower alkoxycarbonyl group includes, for example, a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a neopentyloxycarbonyl group, a tert-pentyloxycarbonyl group, a hexyloxycarbonyl group and an isohexyloxycarbonyl group.

A carbamoyl group which may be substituted means an unsubstituted carbamoyl group or a carbamoyl group substituted with a carboxy lower alkyl group (or a lower alkyl ester form thereof). The carbamoyl group substituted with a carboxy lower alkyl group (or a lower alkyl ester form thereof) includes, for example, a carboxymethylcarbamoyl group, a 1-carboxyethylcarbamoyl group, a 2-carboxyethylcarbamoyl group, a 1-carboxypropylcarbamoyl group, a 2-carboxypropylcarbamoyl group, a 3-carboxypropylcarbamoyl group, a 2-carboxy-1-methylethylcarbamoyl group, a 1-carboxy-1-methylethylcarbamoyl group, a methoxycarbonylmethylcarbamoyl group, an ethoxycarbonylmethylcarbamoyl group, a propoxycarbonylmethylcarbamoyl group, an isopropoxycarbonylmethylcarbamoyl group, a butoxycarbonylmethylcarbamoyl group and a tert-butoxycarbonylmethylcarbamoyl group.

The 3- to 7-membered nitrogen-containing heterocyclic group includes, for example, an aziridinyl group, an azetidinyl group, a pyrrolidinyl group, a piperidinyl group, a hexahydroazepinyl group, a pyrrolidinyl group, an imidazolyl group, a pyrazolyl group, a piperazinyl group, a pyridyl group, a pyrazinyl group, a pyrimidyl group, a triazinyl group and a tetrazolyl group.

The compounds of the present invention have at least an asymmetric carbon atom and exist in isomers (optical isomers, optically active forms, and racemates).

Accordingly, the compounds of the present invention include an isolated isomer of these isomers or a mixture thereof.

Also, the compounds of the present invention may be isolated, in some instances, as hydrates, solvates with a solvent such as ethanol, or different crystalline forms due to polymorphism, and the present invention includes these compounds.

The compound of the present invention forms a salt with an acid or a base. A salt with a pharmaceutically acceptable acid includes a mineral acid salt such as hydrochloride, hydrobromide, hydroiodide, nitrate, sulfate and phosphate, and an acid addition salt with an organic acid such as formate, acetate, propionate, oxalate, malonate, succinate, fumarate, lactate, malate, citrate, tartrate, carbonate, picrate, methanesulfonate, ethanesulfonate and glutamate. A salt with a pharmaceutically acceptable base include a salt with an inorganic base such as a sodium salt, a potassium salt, a magnesium salt and a calcium salt, and a salt with an organic base such as dimethylamine, diethylamine, or an aminosaccharide such as glucamine.

## Preparation Method

The compounds (I) of the present invention can be prepared by applying various synthetic methods. Typical preparation processes thereof are illustrated below.

First Preparation Process

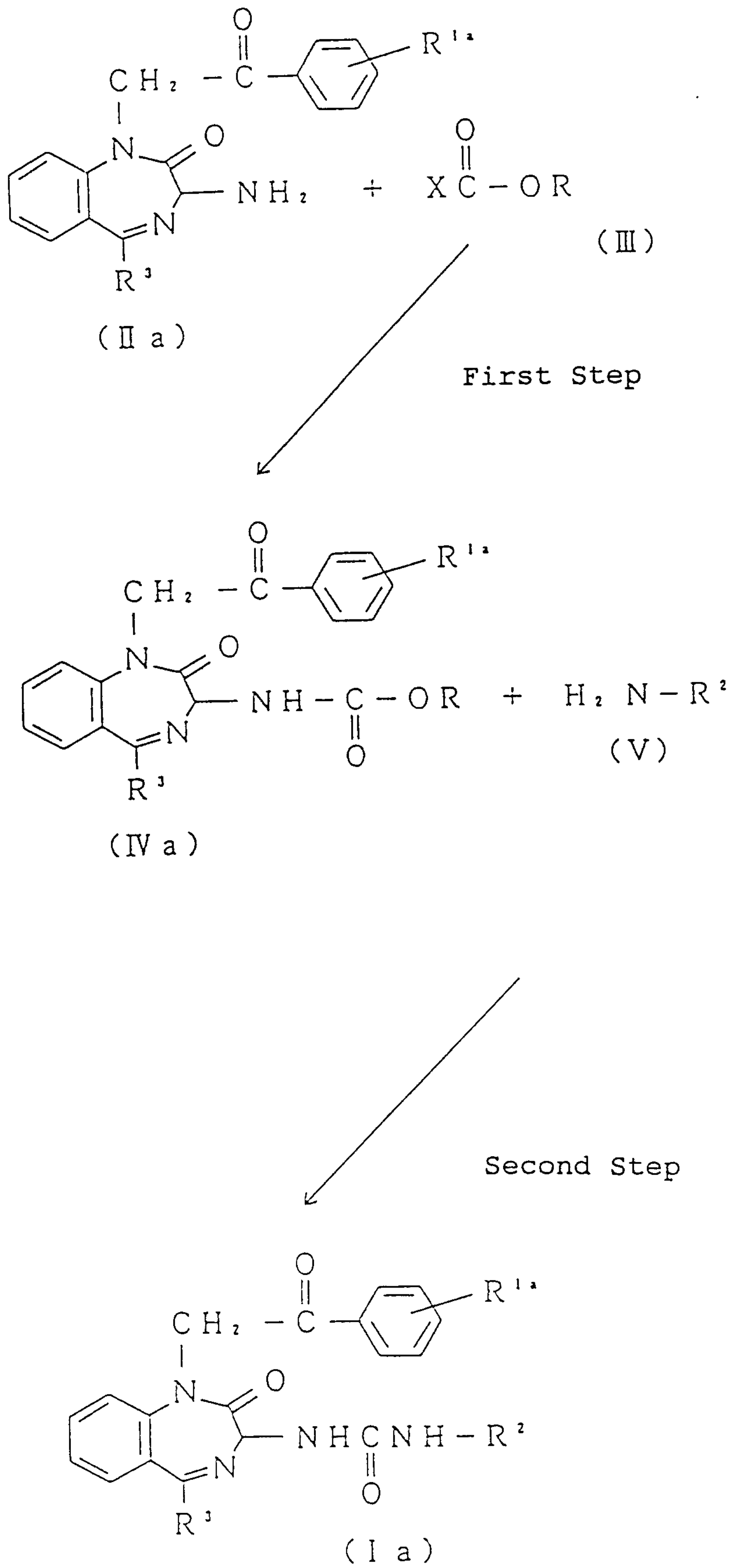


(IIa) (III) (IVa) (V) (Ia)

wherein $R^{1a}$ represents a hydrogen atom or a lower alkyl group, $R^2$ and $R^3$ have the same meanings as above, R represents a lower alkyl group, a phenyl group or a 4-nitrophenyl group, and X represents a halogen atom.

The compound (Ia) of the present invention can be obtained by reacting an amine represented by the general formula (IIa) with a reaction equivalent amount of a haloformate represented by the general formula (III) to produce a carbamic acid ester represented by the general formula (IVa) (a first step), and further reacting the resulting ester with a reaction equivalent amount of an amine represented by the general formula (V) (a second step).

Examples of the haloformate represented by the general formula (III) include isobutylcarbonic acid chloride, methylcarbonic acid chloride, ethylcarbonic acid bromide, phenylcarbonic acid chloride and 4-nitrophenylcarbonic acid chloride. The reaction may be advantageously carried out in the presence of a base for promoting the reaction such as potassium carbonate, sodium carbonate, sodium hydroxide, potassium hydroxide, triethylamine and N,N-dimethylaniline.

As a reaction solvent, any of inert solvents such as N,N-dimethylformamide, chloroform, benzene, toluene, xylene, dioxane, diethyl ether, tetrahydrofuran, chloroform, dichloromethane and dichloroethane may be used. The reaction temperature is adjusted to a temperature from a temperature under cooling to room temperature in the reaction between the amine represented by the general formula (IIa) and the haloformate represented by the general formula (III), and from room temperature to a temperature under heating in the reaction between the carbamic acid ester represented by the general formula (IVa) obtained above and the amine represented by the general formula (V).

Second Preparation Process

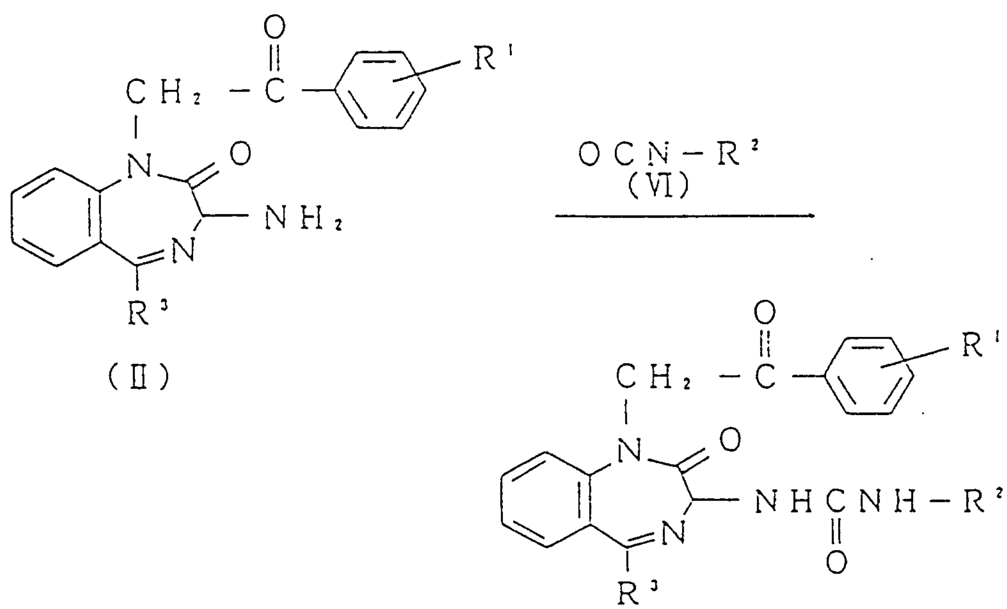


(II)

(I)

wherein $R^1$, $R^2$ and $R^3$ have the same meanings as defined above.

The compound (I) of the present invention can be obtained by reacting an amine represented by the general formula (II) with a reaction equivalent amount of an isocyanate represented by the general formula (VI) which is commercially available or which can be prepared just before use.

The reaction can be conducted in a solvent inert to the reaction such as N,N-dimethylformamide, pyridine, benzene, toluene, dioxane, tetrahydrofuran, diethyl ether, chloroform, dichloromethane, dichloroethane and n-hexane, with stirring generally at room temperature or under heating.

Other Preparation Processes

Other methods for preparing the object compounds include the following procedures in which the substituents in the general formula (I) are interconverted:

1. The compound of the present invention having a carboxyl group for $R^2$ can be prepared by dissolving a corresponding lower alkyl ester in a solvent such as methanol, ethanol and dichloromethane, adding a basic aqueous solution such as a sodium hydroxide aqueous solution and a potassium hydroxide aqueous solution to the solution and, after stirring at room temperature or under warming, adding an acidic aqueous solution such as hydrochloric acid and sulfuric acid to the reaction solution.
2. The compound of the present invention having a carbamoyl group for $R^2$ can be prepared by amidation of a corresponding compound having a carboxyl group in a conventional manner. This amidation can be carried out by dissolving the carboxylic acid in an inert solvent such as benzene, chloroform and tetrahydrofuran, adding a reaction equivalent amount of a haloformic acid ester and a base to the solution to form a mixed acid anhydride, and adding a reaction equivalent amount or an excess amount of an amine, followed by stirring at room temperature or under warming.
3. The compound of the present invention having an amino group for $R^2$ can be prepared by hydrolyzing a corresponding acylamine. The hydrolysis can be performed by suspending the amide in an inert solvent such as acetone, benzene, chloroform and tetrahydrofuran, and adding a reaction equivalent amount of an acid such as hydrochloric acid, sulfuric acid and nitric acid to the suspension, followed by stirring at room temperature or under warming.

The compound of the present invention prepared in these processes is isolated in a free form or as a salt thereof and then purified.

The isolation and purification can be conducted by applying ordinary chemical procedures such as extraction, concentration, distillation, crystallization, filtration, recrystallization and various types of chromatography.

Also, a racemic mixture can be converted into a stereochemically pure isomer thereof by using an appropriate starting compound, or by using a usual racemate resolution method, for example, by a method comprising the formation of a diastereomeric salt with a usual optically active acid (e.g., tartaric acid) and subjecting the salt to an optical resolution.

POSSIBILITY OF UTILIZATION IN INDUSTRY

The compounds of the present invention have a markedly excellent CCK-B receptor antagonistic activity, a gastrin receptor antagonistic activity and a gastric acid secretion inhibitory activity. The compounds of the present invention are also characterized in that they exhibit a remarkable gastric acid secretion inhibitory activity by oral administration. These activities are described hereinafter, together with the methods for measurement thereof.

(1) Binding Activity to CCK-B Receptor

Method of Measurement

About 100 conscious SD rats were sacrificed by decapitation, and immediately thereafter the whole brain was extracted. The whole brain was homogenized using a Teflon-glass homogenizer in a 0.32M sucrose aqueous solution in a volume of 10 times the whole brain. The resulting homogenate was centrifuged in a cooled centrifugator at 900 × g for 10 minutes, and the supernatant was further centrifuged at 11,500 × g for 15 minutes. The resulting sediment was suspended in a 0.08% Triton X-100 and a 50 mM Tris-HCl buffering solution (pH 7.4). After allowing the suspension to stand for 30 minutes, the suspension was again centrifuged at 11,500 × g for 15 minutes, and the sediment was washed twice with each of 5 mM and 50 mM Tris-HCl buffers by centrifuging operations. The resulting sediment was suspended in a 50 mM Tris-HCl buffer, and freezed at -80 °C for storage as a membrane sample.

After thawing the membrane sample at room temperature, it was diluted with a 10 mM HEPES buffer (130 mM NaCl, 5 mM $MgCl_2$•1mM EGTA, 0.25 mg/ml bacitracin, pH 6.5) and, after incubating at 25 °C for 120 minutes in the presence of [$^{125}$I]BH.CCK-8, B/F separation was performed by suction filtration. The non-specific binding was determined in the presence of 1 $\mu$M of CCK-8. The amount of the labelled ligand bonded to the receptor was measured by a $\gamma$-counter. A Ki (CCK-B) value was determined from a displacement curve of the test compound by taking the maximum binding amount of the labelled ligand to the receptor as 100%.

The $IC_{50}$ values of the compounds of the present invention were from 0.03 to 10 nM. Specific examples thereof are shown below.

| | CCK-B Binding $IC_{50}$ (nM) |
|---|---|
| Compound described in Example 281 of U.S. Patent 4,820,834 | 29.0 |
| Compound of Example 3 | 0.17 |
| Compound of Example 21 | 0.16 |
| Compound of Example 35 | 2.14 |

(2) Inhibitory Activity on Gastric Acid Secretion Stimulated by Pentagastrin

(a) Inhibitory Activity in Rats by Intravenous Administration

(i) Method of Measurement: A cannula was inserted into a trachea of rats anesthetized with urethane (1.25 g/kg intraperitoneal administration) and abdomen was incised to expose stomach and duodenum. After ligating the cardiac portion, a polyethylene cannula was set in the forestomach portion. Further, a small incision was given to the duodenum, and a polyethylene cannula was inserted from the incised portion to a direction of the stomach inside. In order to fix the cannula, the pylorus portion was ligated.

A physiological saline (adjusted to pH 7.0) was perfused from forestomach portion to the pylorus portion at a rate of 3 ml/minute. The gastric acid secretion was measured by continuously titrating the perfusate by means of a pH-stat (AUT-201, produced by Toa Electronics Ltd.). The continuous titration was performed by titrating with a 25 mM sodium hydroxide solution using a titration end point of pH 7.0. The results were determined as a gastric acid analysis at 10 minutes intervals (μEq/10 minutes). Then, pentagastrin was intravenously administered (15 μg/kg/hour). After injection of pentagastrin, the acid secretion was increased, and 60 minutes after the injection, the acid secretion reached to its maximum value and stabilized. Thereafter, a test drug was intravenously administered, and the gastric acid secretion was measured. From the gastric acid secretion amount after the test drug injection, a gastric acid secretion inhibitory ratio was calculated by taking the maximum value after the pentagastrin injection as 100%. In this test, two species of rats, i.e., Wistar species rats and SD species rats, were used as test animals, but the embodiments obtained by using the SD species rats are described below.

The compounds of the present invention show from 9 to 89% gastric acid secretion inhibitory effects by the administration at a dose of 0.1 μmol/kg.

The embodiments are shown below.

| | Gastric Acid Secretion Inhibitory Ratio/Dose |
|---|---|
| Compound described in Example 281 of U.S. Patent 4,820,834 | 15% (1 μmol/kg) |
| Compound of Example 3 | 63% (0.1 μmol/kg) |
| Compound of Example 21 | 74% (0.1 μmol/kg) |
| Compound of Example 35 | 76% (0.1 μmol/kg) |

(ii) Method of Measurement: Male beagle dogs (7 to 12 kg) fasted overnight was used and subjected to peritoneotomy under GOF anesthesia. After amputating the corpus ventriculi portion parallel to the curvatura ventriculi major, a metal pouch was retained in the suture-closed epigastric region to prepare Heidenhain pouch dogs. The dogs under healthy conditions after two months from the operation were presented to the experiments. Each of the pouch dogs was used at one week intervals.

Pentagastrin was continuously infused (8 μg/kg/hr) into 3 to 5 Heidenhain pouch dogs fasted for 18 hours, and the gastric juice naturally dropped from the pouch was collected at a 15 minute interval. The acid concentration in the gastric juice was calculated from the amount of a 0.05N NaOH required for titrating up to pH of 7.0 using an automatic titration apparatus (Comtite-7, produced by Hiranuma Sangyo), and the product with the amount of the gastric juice was referred to as an acid secretion amount.

Three hours after commencement of the continuous infusion of pentagastrin, a test drug was orally administered, and an inhibitory ratio to the gastric acid secretion at 15 minute intervals was determined to evaluate the effects of the drug.

| | Gastric Acid Secretion Inhibitory Ratio/Dose |
|---|---|
| Compound described in Example 281 of U.S. Patent 4,820,834 | 0% (100 µmol/kg) |
| Compound of Example 3 | 57% (10 µmol/kg) |
| Compound of Example 21 | 90% (10 µmol/kg) |
| Compound of Example 35 | 83% (1 µmol/kg) |

From the above experiments, the compounds of the present invention have a CCK-B receptor antagonistic activity and a pentagastrin stimulated gastric acid secretion inhibitory activity and, therefore, are useful for the treatment and prophylaxis of the diseases relating to the CCK-B receptor and the gastrin receptor.

Examples of such diseases include digestive disorders such as gastric ulcer, duodenum ulcer, gastritis, reflux esophagitis, Zollinger-Ellison syndrome and gastrin sensitive pancreas, appetite controlling system disorders, and central nervous system disorders such as pain and anxiety.

The pharmaceutical composition containing, as an active ingredient, one or more of the compounds (I), pharmaceutically acceptable salts or hydrates thereof can be prepared by using carriers or excipients and other additives generally used for pharmaceutical preparations. The carriers and excipients used for the preparations may be either solid or liquid, and examples thereof include lactose, magnesium stearate, starch, talc, gelatin, agar, pectin, gum arabic, olive oil, sesame oil, cacao butter, ethylene glycol as well as other materials ordinary used.

The administration can be oral administration in the form of tablets, pills, capsules, granules, powders or liquid preparations, or intravenous or intramuscular administration in the form of injectable preparations, or parenteral administration in the form of a suppositories, transdermal preparations and transmucosal preparations, and may be administered systemically or locally.

The dose varies depending upon the age, body weight, conditions, therapeutic effects, methods of administration, treating time, etc. but, in an individual adult, is generally in the range of from 0.1 to 100 mg, preferably from 1 to 50 mg per day by oral administration in a single dose or divided into several doses, or in the range of from 0.05 mg to 50 mg per day by intravenous administration in a single dose or divided into several doses or by continuous administration for a period in the range of from 1 to 24 hours daily. Of course, since the dose varies depending upon the various conditions as described above, a dose lower than the above range may sometimes be sufficient.

## BEST MODE FOR CARRYING OUT THE INVENTION

Preparation examples of preparations using the compound of the present invention are described hereinafter.

| Preparation Example (Tablets) | | |
|---|---|---|
| Composition | 20 mg Tablet | 40 mg Tablet |
| Compound of Example | 20 mg | 40 mg |
| Lactose | 73.4 | 80 |
| Corn starch | 18 | 20 |
| Hydroxypropylcellulose | 4 | 5 |
| Carboxymethylcellulose calcium | 4 | 4.2 |
| Magnesium stearate | 0.6 | 0.8 |
| Total | 120 mg | 150 mg |

### Preparation Method for 20 mg Tablets

100 g of a compound of Example, 367 g of lactose and 90 g of corn starch were mixed uniformly using a fluidized granular coating machine (produced by Ohkawara Manufacturing Co., Ltd.). Then, 200 g of a 10% hydroxypropylcellulose solution was sprayed to the mixture and granulated. After drying, the granules were passed through a 20 mesh sieve, and 20 g of carboxymethyl cellulose calcium and 3 g of magnesium

stearate were added thereto. The mixture was then compressed into tablets each weighing 120 mg in a rotary tabletting machine (produced by Hata Tekkosho Co., Ltd.) using a die of 7 mm x 8.4 R.

Preparation Method for 40 mg Tablets

140 g of a compound of Example, 280 g of lactose and 70 g of corn starch were mixed uniformly using a fluidized granular coating machine (produced by Ohkawara Manufacturing Co., Ltd.). Then, 175 g of a 10% hydroxypropylcellulose solution was sprayed to the mixture and granulated. After drying, the granules were passed through a 20 mesh sieve, and 14.7 g of carboxymethylcellulose calcium and 2.8 g of magnesium stearate were added thereto. The mixture was then compressed into tablets each weighing 150 mg in a rotary tabletting machine (produced by Hata Tekkosho Co., Ltd.) using a die of 7.5 mm x 9 R.

The present invention is further illustrated by the following examples. Of the starting compounds used in the examples, the preparation examples for the novel compounds are shown in Reference Examples or Examples.

In the following descriptions, MP, MS and NMR stand for a melting point, a mass spectrum data and a nuclear magnetic resonance spectrum, respectively.

REFERENCE EXAMPLE 1

(Starting Compound for Examples 4, 5, 7, 8, 16 and 18)

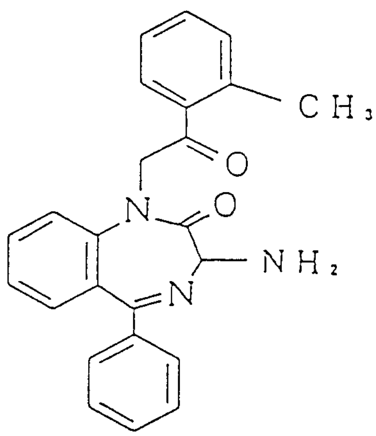

(a) A mixed solution of 29.55 g of sodium hydroxide and 60 ml of water was added to a mixed solution of 12.12 g of 1,3-dihydro-5-phenyl-2H-1,4-benzodiazepin-2-one, 16.40 g of 2-bromo-2'-methylacetophenone, 0.27 g of tricaprylmethylammonium chloride and 180 ml of toluene under ice cooling, followed by stirring at room temperature for 1.5 hour. The organic layer was separated, and the aqueous layer was extracted with 450 ml of toluene. The organic layers were combined, washed successively with 150 ml of water 4 times and a saturated aqueous sodium chloride solution, and dried over anhydrous magnesium sulfate.

The residue obtained by distilling off the solvent was subjected to silica gel column chromatography, and the fractions eluted with a mixed solvent of ethyl acetate-n-hexane (3:2 to 1:1) were recrystallized from a mixed solvent of ethyl acetate-n-hexane to obtain 14.78 g of 1,3-dihydro-1-(2'-methylphenacyl)-5-phenyl-2H-1,4-benzodiazepin-2-one.

MP: 119-121 °C

MS EI (m/z): 368 ($M^+$)

NMR ($CDCl_3$, TMS Internal Standard)

$\delta$: 2.46 (3H, s), 3.93 (1H, d), 4.87 (1H, d), 5.14 (2H, d), 7.05-7.75 (13H, m)

(b) 11.19 g of potassium tert-butoxide was added to a mixed solution of 14.70 g of the compound obtained as above and 205 ml of toluene at -20 °C, followed by stirring for 20 minutes. 7.01 g of isoamyl nitrite was added dropwise thereto over 10 minutes, and the mixture was stirred at -20 to -15 °C for 1.5 hour. The reaction solution was poured into a mixed solution of 410 g of ice water, 20 ml of acetic acid and 410 ml of ethyl acetate and, after stirring one hour, the mixture was separated, and aqueous layer was extracted with 200 ml of ethyl acetate. The organic layers were combined, washed successively with water and a saturated aqueous sodium chloride solution, and dried over anhydrous magnesium sulfate.

The residue obtained by distilling off the solvent was powdered from a mixed solution of ethyl acetate-n-hexane to obtain 12.33 g of 1,3-dihydro-1-(2'-methylphenacyl)-3-oxyimido-5-phenyl-2H-1,4-benzodiazepin-2-one containing a small amount of the starting material. A part of the product was subjected to silica gel column chromatography, and the fraction eluted with a mixed solvent of ethyl acetate-n-hexane (1:1) was recrystallized from a mixed solution of ethyl acetate-n-hexane to obtain a pure product.

MP: 222-227 °C

| Elementary Analysis (for $C_{24}H_{19}N_3O_3$) | | | |
|---|---|---|---|
| | C (%) | H (%) | N (%) |
| Calc'd values: | 72.53 | 4.82 | 10.57 |
| Found values: | 72.37 | 4.91 | 10.32 |

MS EI (m/z): 397 ($M^+$)

NMR (DMSO, TMS Internal Standard)

δ: 2.33 (3H, s), 5.35 (2H, s), 7.10-7.90 (13H, m), 11.08 (1H, s)

(c) A mixed solution of 20.98 g of the compound obtained above, 4.20 g of a 5% ruthenium-carbon powder and 420 ml of methanol was stirred in a pressurized hydrogen gas (8 kg/cm$^2$), at 60 °C for 23 hours. The catalyst was removed from the reaction solution, and 285 ml of acetonitrile and 10 ml of an acetonitrile solution of 7.57 g of (±)-mandelic acid in 100 ml of acetonitrile were added successively to 19.07 g of the residue obtained by distilling off the solvent. After stirring the mixture at room temperature for one hour, the precipitated crystals were separated by filtration to obtain 16.36 g of 3-amino-1,3-dihydro-1-(2'-methylphenacyl)-5-phenyl-2H-1,4-benzodiazepin-2-one mandelate.

MP: 146-150 °C

MS FAB, Pos. (m/z): 384 ($M^+$ + 1)

NMR (DMSO, TMS Internal Standard)

δ: 2.33 (3H, s), 4.64 (1H, s), 4.86 (1H, s), 5.35 (2H, s), 6.22 (4H, br), 7.23-7.85 (18H, m)

The mandelate obtained above was treated with a 0.25N aqueous sodium hydroxide solution to obtain free 3-amino-1,3-dihydro-1-(2'-methylphenacyl)-5-phenyl-2H-1,4-benzodiazepin-2-one.

MS FAB, Pos. (m/z): 384 ($M^+$ + 1)

NMR ($CDCl_3$, TMS Internal Standard)

δ: 2.10 (2H, br, s), 2.44 (3H, s), 4.62 (1H, s), 5.20 (2H, s), 7.10-7.69 (13H, m)

REFERENCE EXAMPLE 2

(Starting Compound for Examples 1, 3, 6, 13 and 14)

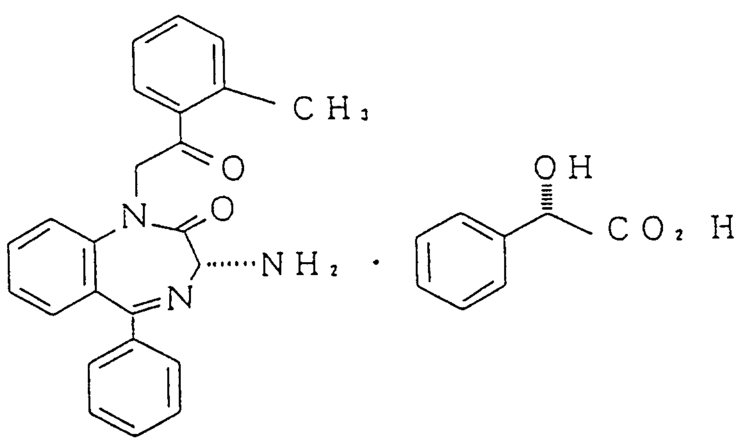

0.98 g of (S)-(+)-mandelic acid was added to 55 ml of an acetonitrile solution of 2.75 g of 3-amino-1,3-dihydro-1-(2'-methylphenacyl)-5-phenyl-2H-1,4-benzodiazepin-2-one in 55 ml of acetonitrile, followed by stirring at room temperature. After 30 minutes, 41 mg of 3,5-dichlorosalicylaldehyde was added thereto, and the mixture was stirred for 18 hours. The precipitated crystals were separated by filtration and washed with

15 ml of acetonitrile to obtain 2.94 g of (R)-3-amino-1,3-dihydro-1-(2'-methylphenacyl)-5-phenyl-2H-1,4-benzodiazepin-2-one•(S)-mandelate.

Specific Rotation $[\alpha]_D^{20}$ = +152.5° (c=1.00, MeOH)

MP: 157-160°C

| Elementary Analysis (for $C_{24}H_{21}N_3O_2 \cdot C_8H_8O_3$) | | | |
|---|---|---|---|
| | C (%) | H (%) | N (%) |
| Calc'd values: | 71.76 | 5.46 | 7.85 |
| Found values: | 71.64 | 5.49 | 7.79 |

REFERENCE EXAMPLE 3

(Starting Compound for Example 17)

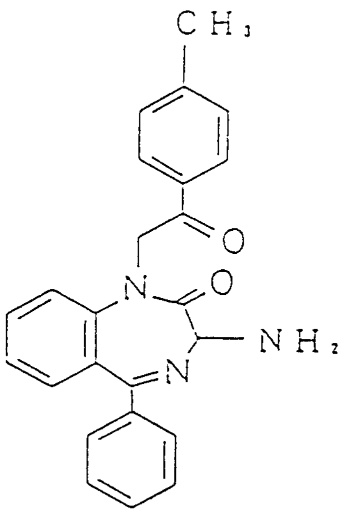

The compound of Reference Example 3 was obtained in the same manner as in Reference Example 1.

(a) Compound Produced: 1,3-Dihydro-1-(4'-methylphenacyl)-5-phenyl-2H-1,4-benzodiazepin-2-one

MS EI (m/z): 368 ($M^+$)

NMR ($CDCl_3$, TMS Internal Standard)

δ: 2.33 (3H, s), 3.93 (1H, d), 4.88 (1H, d), 5.26 (2H, d), 7.06-7.85 (13H, m)

(b) Compound Produced: 1,3-Dihydro-1-(4'-methylphenacyl)-3-oxyimido-5-phenyl-2H-1,4-benzodiazepin-2-one

Starting Compound: 1,3-Dihydro-1-(4'-methylphenacyl)-5-phenyl-2H-1,4-benzodiazepin-2-one

MP: 221-224°C

| Elementary Analysis (for $C_{24}H_{19}N_3O_3$) | | | |
|---|---|---|---|
| | C (%) | H (%) | N (%) |
| Calc'd values: | 72.53 | 4.82 | 10.57 |
| Found values: | 72.45 | 4.91 | 10.39 |

MS EI (m/z): 397 ($M^+$)

NMR (DMSO, TMS Internal Standard)

δ: 2.36 (3H, s), 5.52 (2H, d), 7.24-7.94 (13H, m), 11.07 (1H, s)

(c) Compound Produced: 3-Amino-1,3-dihydro-1-(4'-methylphenacyl)-5-phenyl-2H-1,4-benzodiazepin-2-one

Starting Compound: 1,3-Dihydro-1-(4'-methylphenacyl)-3-oxyimide-5-phenyl-2H-1,4-benzodiazepin-2-one

MS FAB, Pos. (m/z): 384 ($M^+$ + 1)

NMR ($CDCl_3$, TMS Internal Standard)

δ: 2.00 (2H, br, s), 2.34 (3H, s), 4.66 (1H, s), 5.34 (2H, s), 7.16-7.88 (13H, m)

REFERENCE EXAMPLE 4

(Starting Compound for Examples 21, 22, 25, 28 and 29)

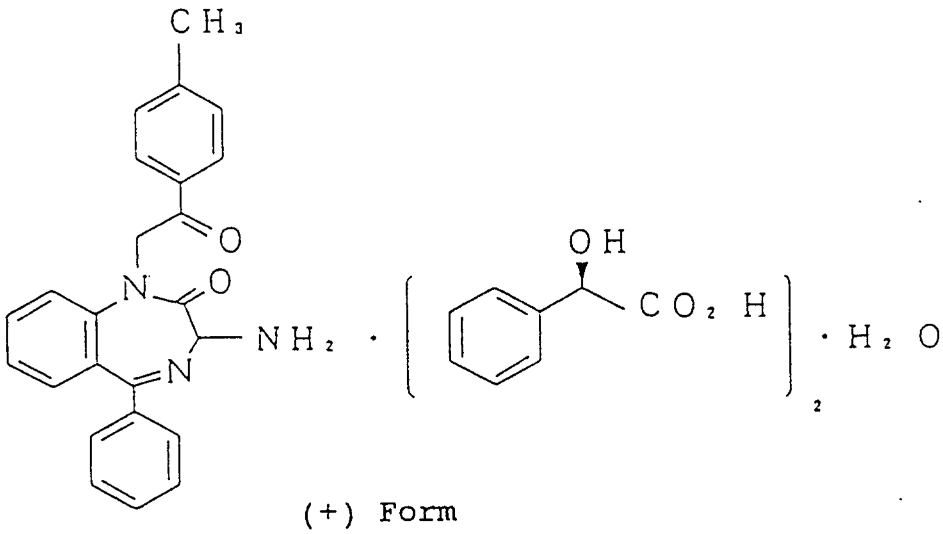

(+) Form

Diastereomer A

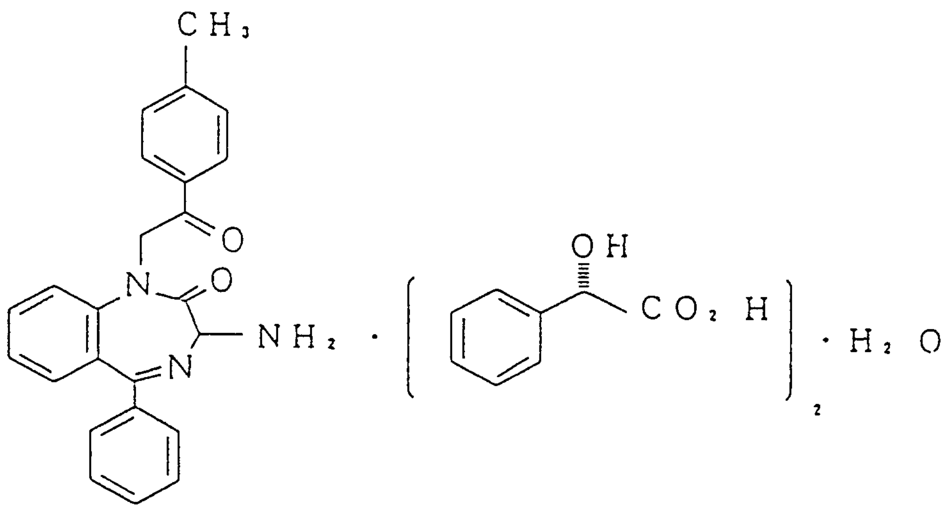

(-) Form

Diastereomer B

(a) 172 mg of diphenylphosphoryl azide and 63 mg of triethylamine were added to a mixed solution of 200 mg of 3-amino-1,3-dihydro-1-(4'-methylphenacyl)-5-phenyl-2H-1,4-benzodiazepin-2-one, 1.5 ml of N,N-dimethylformamide and 116 mg of N-(t-butoxycarbonyl)-D-phenylalanine under ice cooling, followed by stirring for one hour under ice cooling and overnight at room temperature. 20 ml of a 10% aqueous citric acid solution was added to the reaction solution, and the mixture was extracted with 50 ml of ethyl acetate-toluene (2:1). The extract was washed successively with a saturated aqueous sodium bicarbonate solution, water and a saturated aqueous sodium chloride solution and, after drying over anhydrous

magnesium sulfate, the solvent was distilled off under reduced pressure. The residue was crystallized from n-hexane, and the crystals were separated by filtration and washed with n-hexane to obtain 324 mg of 1,1-dimethylethyl [(R)-2-[[2,3-dihydro-1-(4'-methylphenacyl)-2-oxo-5-phenyl-1H-1,4-benzodiazepin-3-yl]amino]-2-oxo-1-(phenylmethyl)ethyl]carbamate.

MS FAB, Pos. (m/z): 631 ($M^+$ + 1)

NMR ($CDCl_3$, TMS Internal Standard)

δ: 1.40 (9H, s), 2.33 (3H, s), 3.00-3.30 (3H, m), 5.20-5.40 (2H, m), 5.59-5.62 (1H, m), 7.10-7.83 (20H, m)

(b) 1.2 ml of a 4N hydrochloric acid-ethyl acetate solution was added to 300 mg of 1,1-dimethylethyl [-(R)-2-[[2,3-dihydro-1-(4'-methylphenacyl)-2-oxo-5-phenyl-1H-1,4-benzodiazepin-3-yl]amino]-2-oxo-1-(phenylmethyl)ethyl]carbamate under ice cooling, and the mixture was stirred for one hour under ice cooling. The reaction solution was adjusted to pH 9 with a 1N aqueous sodium hydroxide solution, and extracted twice with 10 ml of ethyl acetate. The extract was washed with water and a saturated aqueous sodium chloride solution and, after drying over anhydrous sodium sulfate, the solvent was distilled off under reduced pressure. The residue was subjected to silica gel chromatography, and elution was carried out with ethyl acetate to obtain 89 mg of diastereomer A of 2(R)-amino-N-[2,3-dihydro-1-(4'-methylphenacyl)-2-oxo-5-phenyl-1H-1,4-benzodiazepin-3-yl]benzenepropaneamide (Rf value, 0.58) (hereinafter called Compound (b)-A) and 53 mg of diastereomer B of the above compound (Rf value, 0.45) (hereinafter called Compound (b)-B).

Compound (b)-A

MS FAB, Pos. (m/z): 531 ($M^+$ + 1)

NMR ($CDCl_3$, TMS Internal Standard)

δ: 2.32 (3H, s), 2.82 (1H, dd), 3.38 (1H, dd), 3.71 (1H, dd), 5.28, 5.35 (each 1H, each d), 5.70 (1H, d), 7.10-7.90 (20H, m), 8.90 (1H, d)

Rf value: 0.58, developing solvent, ethyl acetate

Compound (b)-B

MS FAB, Pos. (m/z): 531 ($M^+$ + 1)

NMR ($CDCl_3$, TMS Internal Standard)

δ: 2.32 (3H, s), 2.70 (1H, dd), 3.37 (1H, dd), 3.75 (1H, dd), 5.26, 5.38 (each 1H, each d), 5.68 (1H, d), 7.10-7.90 (20H, m), 8.98 (1H, d)

Rf value: 0.45, developing solvent, ethyl acetate

(c) A mixed solution of 25 mg of phenyl isothiocyanate and 0.5 ml of dichloromethane was added dropwise to a mixed solution of 89 mg of Compound (b)-A obtained in the above-described Step (b), followed by stirring at room temperature for 15 hours. The solvent of the reaction solution was distilled off, n-hexane was added to the residue, and the precipitated crystals were separated by filtration to obtain 91 mg of 1-[[1-[2,3-dihydro-1-(4'-methylphenacyl)-2-oxo-5-phenyl-1H-1,4-benzodiazepin-3-yl]-amino-1-oxo-3-phenyl]prop-2(R)-yl]-3-phenylthiourea (hereinafter called Compound (c)-A).

On the other hand, in the same manner as described above but using 53 mg of Compound (b)-B obtained in the above-described Step (b), 60 mg of 1-[[1-[2,3-dihydro-1-(4'-methylphenacyl)-2-oxo-5-phenyl-1H-1,4-benzodiazepin-3-yl]amino-1-oxo-3-phenyl]prop-2(R)-yl]-3-phenylthiourea (Compound (c)-B) was obtained.

Compound (c)-A

MS FAB, Pos. (m/z): 666 ($M^+$ + 1)

NMR ($CDCl_3$, TMS Internal Standard)

δ: 2.33 (3H, s), 3.34-3.45 (2H, m), 5.23, 5.34 (each 1H, each d), 5.41 (1H, d), 5.60 (1H, d), 6.80-8.00 (26H, m)

Compound (c)-B

MS FAB, Pos. (m/z): 666 ($M^+$ + 1)

NMR ($CDCl_3$, TMS Internal Standard)

δ: 2.33 (3H, s), 3.35-3.42 (2H, m), 5.20, 5.37 (each 1H, each d), 5.37 (1H, d), 5.60 (1H, d), 6.80-7.95 (26H, m)

(d) 0.15 ml of trifluoroacetic acid was added to 83 mg of the above-described Compound (c)-A, followed by stirring at room temperature for 3 hours. The solvent of the reaction solution was distilled off, the residue was subjected to silica gel column chromatography, and elution was carried out with dichloromethane:methanol = 20:1. The eluate was dissolved in dichloromethane and washed with a 1N aqueous sodium hydroxide solution and water. After drying over anhydrous magnesium sulfate, the solvent was distilled off under reduced pressure to obtain 29 mg of (+)-3-amino-1,3-dihydro-1-(4'-methylphenacyl)-5-phenyl-2H-1,4-benzodiazepin-2-one (hereinafter called Compound (d)-A).

On the other hand, in the same manner as described above but using 60 mg of Compound (c)-B obtained in the above-described Step (c), 19 mg of (-)-3-amino-1,3-dihydro-1-(4'-methylphenacyl)-5-phenyl-2H-1,4-benzodiazepin-2-one (hereinafter called Compound (d)-B) was obtained.

Compound (d)-A

MS FAB, Pos. (m/z): 384 ($M^+$ + 1)

NMR ($CDCl_3$, TMS Internal Standard)

δ: 2.33 (3H, s), 4.63 (1H, s), 5.31 (2H, s), 7.14-7.84 (13H, m)

Compound (d)-B

MS FAB, Pos. (m/z): 384 ($M^+$ + 1)

NMR ($CDCl_3$, TMS Internal Standard)

δ: 2.33 (3H, s), 4.63 (1H, s), 5.31 (2H, s), 7.14-7.84 (13H, m)

(e) A suspension of 15 mg of the above-described Compound (d)-A, 10 mg of (R)-(-)-mandelic acid and 1.5 ml of aqueous benzene was heated for dissolution, followed by allowing to cool. The precipitated crystals were separated by filtration to obtain 15 mg of (+)-3-amino-1,3-dihydro-1-(4'-methylphenacyl)-5-phenyl-2H-1,4-benzodiazepin-2-one•2[(R)-mandelate]•monohydrate (Diastereomer A, a starting compound of Example 18).

On the other hand, in the same manner as described above but using 9 mg of the above-described Compound (d)-B and 8 mg of (S)-(+)-mandelic acid, 8 mg of (-)-3-amino-1,3-dihydro-1-(4'-methylphenacyl)-5-phenyl-2H-1,4-benzodiazepin-2-one•2[(S)-mandelate]•monohydrate (Diastereomer B, a starting compound of Example 19) was obtained.

Diastereomer A

Specific Rotation $[\alpha]_D$ = +47.96° (c = 1.03, MeOH)

| Elementary Analysis (for $C_{24}H_{21}N_3O_2 \cdot 2C_8H_8O_3 \cdot H_2O$) | | | |
|---|---|---|---|
| | C (%) | H (%) | N (%) |
| Calc'd values: | 68.07 | 5.57 | 5.95 |
| Found values: | 68.23 | 5.33 | 5.96 |

Diastereomer B

Specific Rotation $[\alpha]_D$ = -50.68° (c = 1.10, MeOH)

NMR (DMSO, TMS Internal Standard)

δ: 2.34 (3H, s), 4.89 (1H, s), 5.08 (2H, s), 5.25-5.40 (2H, m), 7.10-7.85 (18H, m)

(f) A suspension of 2.16 g of 3-amino-1,3-dihydro-1-(4'-methylphenacyl)-5-phenyl-2H-1,4-benzodiazepin-2-one, 1.71 g of (R)-(-)-mandelic acid, 32 mg of 3,5-dichlorosalicylaldehyde and 20 ml of aqueous benzene was heated for dissolution, followed by allowing to cool at room temperature.

After adding a small amount of Diastereomer A obtained in the above-described (e), the mixture was stirred at room temperature for 3 days. The precipitated crystals were separated by filtration to obtain 3.00 g of (+)-3-amino-1,3-dihydro-1-(4'-methylphenacyl)-5-phenyl-2H-1,4-benzodiazepin-2-one•2[(R)-mandelate]•monohydrate. The specific rotation and the nuclear magnetic resonance spectrum (NMR) were completely in agreement with those of Diastereomer A of (e) described above.

In the same manner as described above but using 200 mg of 3-amino-1,3-dihydro-1-(4'-methylphenacyl)-5-phenyl-2H-1,4-benzodiazepin-2-one, 3.159 mg of (S)-(+)-mandelic acid and 3 mg of 3,5-dichlorosalicylaldehyde, 226 mg of (-)-3-amino-1,3-dihydro-1-(4-methylphenacyl)-5-phenyl-2H-1,4-benzodiazepin-2-one•2[(S)-mandelate]•monohydrate was obtained. The physicochemical properties of the product were completely in agreement with those of Diastereomer B of (e) described above.

REFERENCE EXAMPLE 5
(Starting Compound of Example 27)

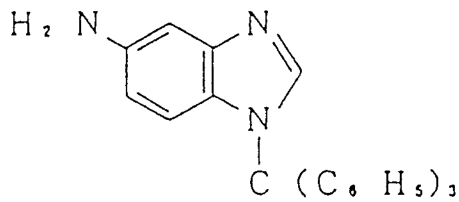

(a) 1 g of 5-nitrobenzimidazole was dissolved in 10 ml of N,N-dimethylformamide, and 0.85 ml of triethylamine and 1.71 g of triphenylmethyl chloride were added thereto, followed by stirring at room temperature for 18 hours. 10 ml of a saturated aqueous sodium bicarbonate solution and 30 ml of chloromethane were added to the reaction solution, and the organic layer was separated. The organic layer was washed successively with water and a saturated aqueous sodium chloride solution, and dried over anhydrous magnesium sulfate. The solvent was distilled off, and the resulting residue was subjected to silica gel column chromatography, and 2.47 g of 5-nitro-1-triphenylmethylbenzimidazole was obtained from the fraction eluted with a mixed solution of dichloromethane-methanol (30:1).

(b) 1.5 g of 5-nitro-1-triphenylmethylbenzimidazole was dissolved in 45 ml of ethanol, and 5 ml of an aqueous solution of 2 g of sodium hydrosulfide was added thereto, followed by heating for one hour under refluxing. After cooling the reaction solution, the solvent was distilled off, and the resulting residue was subjected to silica gel column chromatography to obtain 163 mg of 5-amino-1-triphenylmethylbenzimidazole from the fraction eluted with a mixed solution of dichloromethane-methanol (30:1).

MS EI (m/z): 375 ($M^+$)

EXAMPLE 1

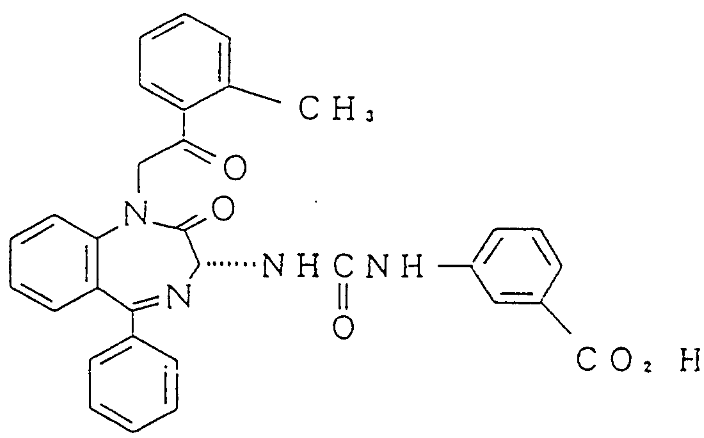

11.59 g of (R)-3-amino-1,3-dihydro-1-(2'-methylphenacyl)-5-phenyl-2H-1,4-benzodiazepin-2-one•(S)-mandelate was dissolved in 20 ml of dichloromethane, and 15 ml of a 0.25N aqueous sodium hydroxide solution was added thereto, followed by stirring for 10 minutes. The organic layer was washed successively with water and a saturated aqueous sodium chloride solution, and dried over anhydrous magnesium sulfate. The solvent was distilled off, and 0.2 ml of triethylamine was added to 10 ml of a tetrahydrofuran solution of the resulting free amine, followed by stirring at 0°C for 30 minutes. 302 mg of 4-nitrophenyl chloroformate was added to the reaction solution, followed by stirring at room temperature for one hour.

The solvent in the reaction solution was distilled off, and the residue was dissolved in 5 ml of N,N-dimethylformamide. 170 mg of 3-aminobenzoic acid and 300 µl of triethylamine were added to the solution, followed by stirring at 45°C for 22 hours. The reaction solution was allowed to cool to room temperature, and 10 ml of ethyl acetate and 10 ml of a 0.3N aqueous hydrochloric acid solution were added thereto. The

organic layer was washed successively with water and a saturated aqueous sodium chloride solution, and dried over anhydrous magnesium sulfate. The solvent was distilled off, and the resulting residue was subjected to silica gel column chromatography. The fraction eluted with a mixture of dichloromethane-methanol (20:1) was recrystallized from dichloromethane-diethyl ether to obtain 266 mg of (R)-[3-[2,3-dihydro-1-(2'-methylphenacyl)-2-oxo-5-phenyl-1H-1,4-benzodiazepin-3-yl]ureido]benzoic acid.

Specific Rotation $[\alpha]_D^{20}$ = +128.3° (c=0.49, DMF)

MP: 226-229°C (decomposition)

| Elementary Analysis (for $C_{32}H_{26}N_4O_5 \cdot 0.2H_2O$) | | | |
|---|---|---|---|
| | C (%) | H (%) | N (%) |
| Calc'd values: | 69.86 | 4.84 | 10.18 |
| Found values: | 69.84 | 4.78 | 10.15 |

MS FAB, Pos. (m/z): 547 ($M^+$ + 1)

EXAMPLE 2

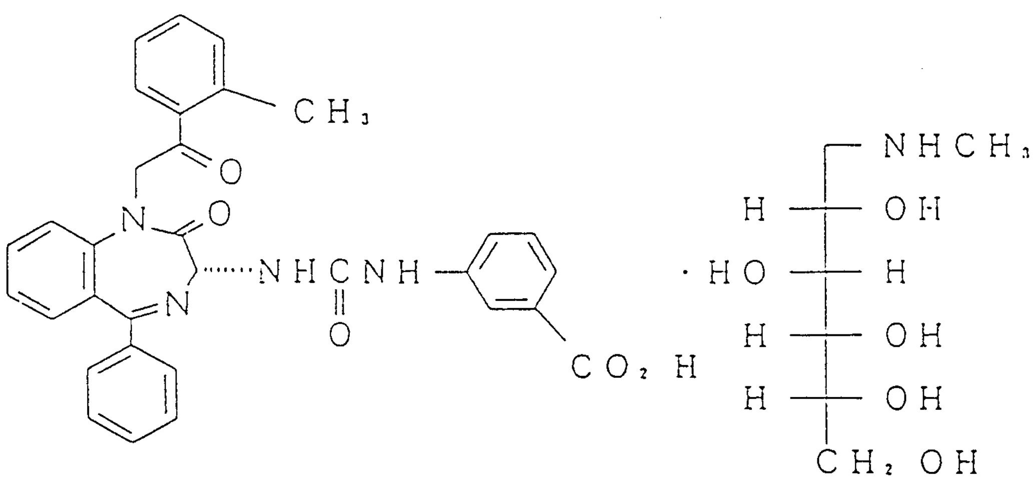

3 ml of methanol was added to 77 mg of (R)-[3-[2,3-dihydro-1-(2'-methylphenacyl)-2-oxo-5-phenyl-1H-1,4-benzodiazepin-3-yl]ureido]benzoic acid, followed by stirring. A solution of 27.5 mg of N-methyl-D-glucamine dissolved in 2 ml of water was added to the above mixed solution at room temperature and, after stirring for 3 hours under ice cooling, the solvent was distilled off. The residue was recrystallized from methanol-diethyl ether to obtain 79 mg of N-methyl-D-glucamine (R)-[3-[2,3-dihydro-1-(2'-methylphenacyl)-2-oxo-5-phenyl-1H-1,4-benzodiazepin-3-yl]ureido]benzoate.

Specific Rotation $[\alpha]_D^{20}$ = +36.4° (c=0.31, MeOH)

MP: 140-143°C

| Elementary Analysis (for $C_{39}H_{43}N_5O_{10} \cdot 2H_2O$) | | | |
|---|---|---|---|
| | C (%) | H (%) | N (%) |
| Calc'd values: | 60.22 | 6.09 | 9.00 |
| Found values: | 60.20 | 5.90 | 9.15 |

EXAMPLE 3

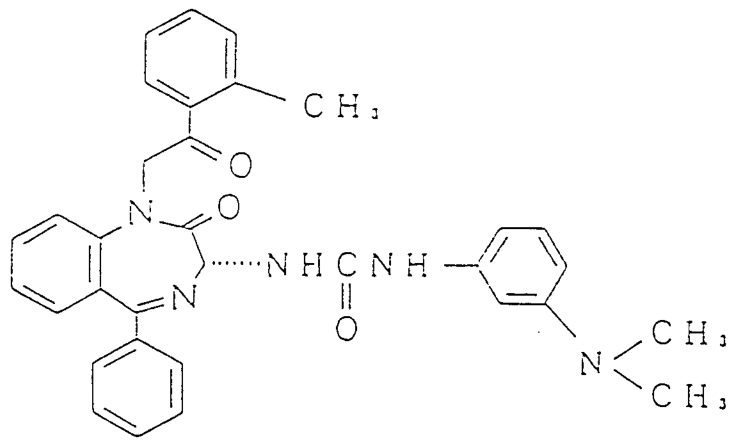

275 mg of diphenylphosphoryl azide and 101 mg of triethylamine were added to a suspension of 165 mg of 3-dimethylaminobenzoic acid in 5 ml of benzene and, after stirring at room temperature for 2 hours, the mixture was heated under refluxing for 3 hours. After allowing the reaction solution to cool to room temperature, 5 ml of a benzene solution of the free amine obtained by treating 150 mg of (R)-3-amino-1,3-dihydro-1-(2'-methylphenacyl)-5-phenyl-2H-1,4-benzodiazepin-2-one•(S)-mandelate with dichloromethane and a 0.25N aqueous sodium hydroxide solution was added to the reaction solution, followed by stirring overnight at room temperature. Then, 10 ml of a saturated aqueous sodium bicarbonate solution and 20 ml of dichloromethane were added thereto, and, after stirring, the organic layer was separated and dried over anhydrous magnesium sulfate. The residue obtained by distilling off the solvent was subjected to silica gel column chromatography, and the fraction eluted with a mixed solution of ethyl acetate-n-hexane (1:1) was recrystallized from diethyl ether to obtain 97 mg of (R)-1-[2,3-dihydro-1-(2'-methylphenacyl)-2-oxo-5-phenyl-1H-1,4-benzodiazepin-3-yl]-3-(3-dimethylaminophenyl)urea.

Specific Rotation $[\alpha]_D^{20}$ = +128° (c=0.54, DMF)

MP: 191-193°C

| Elementary Analysis (for $C_{33}H_{31}N_5O_3$) | | | |
|---|---|---|---|
| | C (%) | H (%) | N (%) |
| Calc'd values: | 72.64 | 5.73 | 12.84 |
| Found values: | 72.42 | 5.77 | 12.86 |

MS FAB, Pos. (m/z): 546 ($M^+$ + 1)

EXAMPLE 4

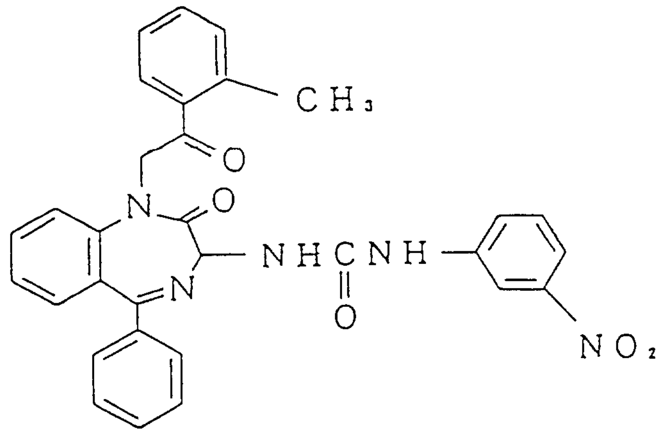

275 mg of diphenylphosphoryl azide and 101 mg of triethylamine were added to 5 ml of a toluene suspension of 167 mg of 3-nitrobenzoic acid, followed by stirring at room temperature for 2 hours. Then, 5 ml of a toluene solution of the free amine obtained by treating 150 mg of 3-amino-1,3-dihydro-1-(2'-methylphenacyl)-5-phenyl-2H-1,4-benzodiazepin-2-one•mandelate with dichloromethane and a 0.25N aqueous sodium hydroxide solution was added thereto, followed by stirring overnight at room temperature.

10 ml of a saturated aqueous sodium bicarbonate and 20 ml of dichloromethane were added to the reaction solution and, after stirring, the organic layer was separated and dried over anhydrous magnesium sulfate. The residue obtained by distilling off the solvent was subjected to silica gel column chromatography, and the fraction eluted with a mixed solution of ethyl acetate-n-hexane (1:1) was recrystallized from diethyl ether-n-hexane to obtain 100 mg of 1-[2,3-dihydro-1-(2'-methylphenacyl)-2-oxo-5-phenyl-1H-1,4-benzodiazepin-3-yl]-3-(3-nitrophenyl)urea.

MP: 222-224 °C

| Elementary Analysis (for $C_{31}H_{25}N_5O_5 \cdot 0.5H_2O$) | | | |
|---|---|---|---|
| | C (%) | H (%) | N (%) |
| Calc'd values: | 66.90 | 4.71 | 12.58 |
| Found values: | 66.98 | 4.65 | 12.72 |

MS FAB, Pos. (m/z): 548 ($M^+$ + 1)

EXAMPLE 5

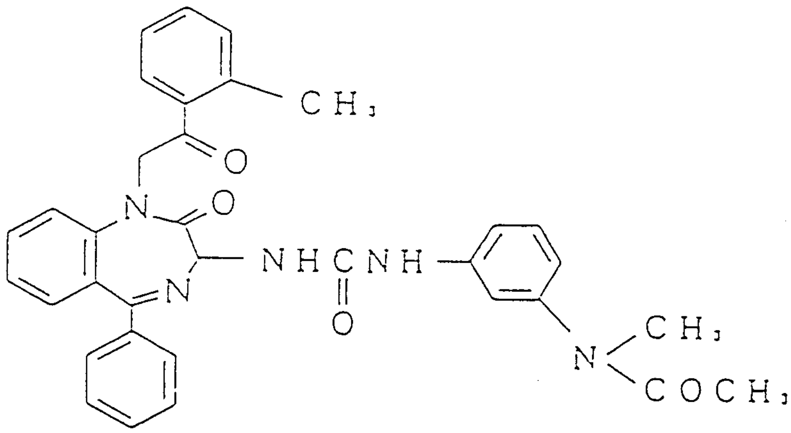

(a) 686 mg of 3-aminobenzoic acid was stirred in 10 ml of acetic anhydride for 5 hours at 100 ° C. After allowing the reaction solution to cool to room temperature, 20 ml of water was added thereto, followed by stirring at room temperature for one hour. The precipitated crystals were separated by filtration and washed with water to obtain 550 mg of 3-acetylaminobenzoic acid.

(b) 150 mg of 60% sodium hydride was added to a 5 ml N,N-dimethylformamide solution of 269 mg of 3-acetylaminobenzoic acid under ice cooling, followed by stirring for 30 minutes. 213 mg of methyl iodide was added to the reaction solution, and the mixture was stirred at a temperature of from 0 ° C to room temperature for 2 hours. 20 ml of 1N hydrochloric acid and 20 ml of dichloromethane were added to the reaction solution and, after stirring, the organic layer was separated and dried over anhydrous magnesium sulfate. The residue obtained by distilling off the solvent was crystallized from n-hexane to obtain 250 mg of 3-(N-acetyl-N-methylamino)benzoic acid.

(c) 138 mg of diphenylphosphoryl azide and 51 mg of triethylamine were added to 5 ml of a toluene suspension of 97 mg of 3-(N-acetyl-N-methylamino)benzoic acid, followed by stirring at room temperature for 2 hours. 5 ml of a toluene solution of the free amine obtained by treating 150 mg of 3-amino-1,3-dihydro-1-(2'-methylphenacyl)-5-phenyl-2H-1,4-benzodiazepin-2-one•mandelate with dichloromethane and a 0.25N aqueous sodium hydroxide solution was added to the reaction solution, and the mixture was stirred at 100 ° C for 30 minutes.

After allowing the reaction solution to cool to room temperature, 20 ml of a saturated aqueous sodium bicarbonate solution and 30 ml of dichloromethane were added thereto, and the organic layer was separated, washed successively with water and a saturated aqueous sodium chloride solution and dried over anhydrous magnesium sulfate. The residue obtained by distilling off the solvent was crystallized from diethyl ether to obtain 150 mg of 1-[3-(N-acetyl-N-methylamino)phenyl]-3-[2,3-dihydro-1-(2'-methylphenacyl)-2-oxo-5-phenyl-1H-1,4-benzodiazepin-3-yl]urea.

MP: 229-231 ° C

| Elementary Analysis (for $C_{34}H_{31}N_5O_4 \cdot 0.25H_2O$) | | | |
|---|---|---|---|
| | C (%) | H (%) | N (%) |
| Calc'd values: | 70.63 | 5.49 | 12.11 |
| Found values: | 70.63 | 5.49 | 12.04 |

MS FAB, Pos. (m/z): 574 ($M^+$ + 1)

EXAMPLE 6

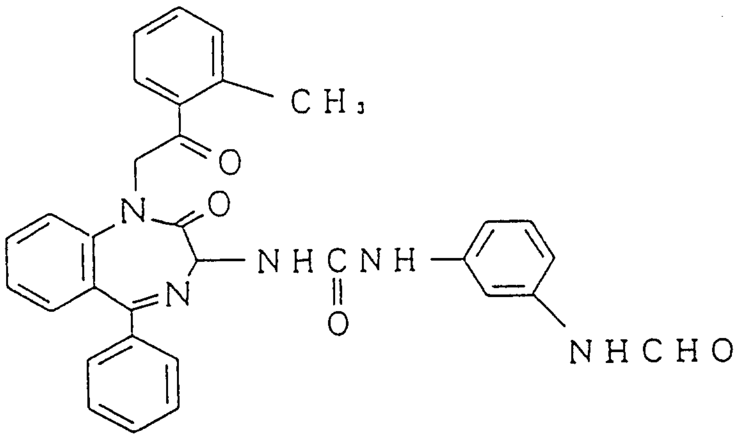

In the same manner as described in Example 3, except for using toluene-N,N-dimethylformamide in place of benzene described in Example 3, the compound of Example 6 was obtained.

Compound Produced:

(R)-1-[2,3-Dihydro-1-(2'-methylphenacyl)-2-oxo-5-phenyl-1H-1,4-benzodiazepin-3-yl]-3-(3-formylaminophenyl)urea Starting Compound: (R)-3-Amino-1,3-dihydro-1-(2'-methylphenacyl)-5-phenyl-2H-1,4-benzodiazepin-2-one• (S)-mandelate and 3-formylaminobenzoic acid

Specific Rotation $[\alpha]_D^{20}$ = +122.7° (c = 0.51, $CH_2Cl_2$)

MP: 155 °C (decomposition)

| Elementary Analysis (for $C_{32}H_{27}N_5O_4 \cdot 0.4H_2O$) | | | |
|---|---|---|---|
| | C (%) | H (%) | N (%) |
| Calc'd values: | 69.53 | 5.07 | 12.67 |
| Found values: | 69.54 | 5.11 | 12.44 |

MS FAB, Pos. (m/z): 564 ($M^+$ + 1)

EXAMPLE 7

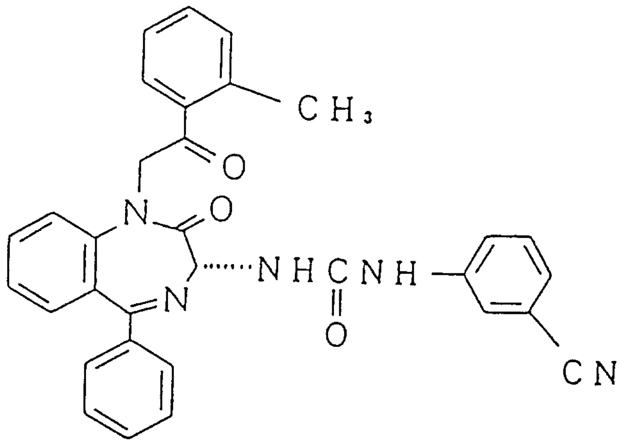

In the same manner as described in Example 3, except for using toluene in place of benzene described in Example 3, the compound of Example 7 was obtained.

Compound Produced:

3-[3-[2,3-Dihydro-1-(2'-methylphenacyl)-2-oxo-5-phenyl-1H-1,4-benzodiazepin-3-yl]ureido]benzonitrile
Starting Compound: 3-Amino-1,3-dihydro-1-(2'-methylphenacyl)-5-phenyl-2H-1,4-benzodiazepin-2-one and 3-cyanobenzoic acid
MP: 238-241 ° C (decomposition)

| Elementary Analysis (for $C_{32}H_{25}N_5O_3$) | | | |
|---|---|---|---|
| | C (%) | H (%) | N (%) |
| Calc'd values: | 72.85 | 4.78 | 13.27 |
| Found values: | 72.94 | 4.84 | 13.29 |

MS FAB, Pos. (m/z): 528 ($M^+$ + 1)

EXAMPLE 8

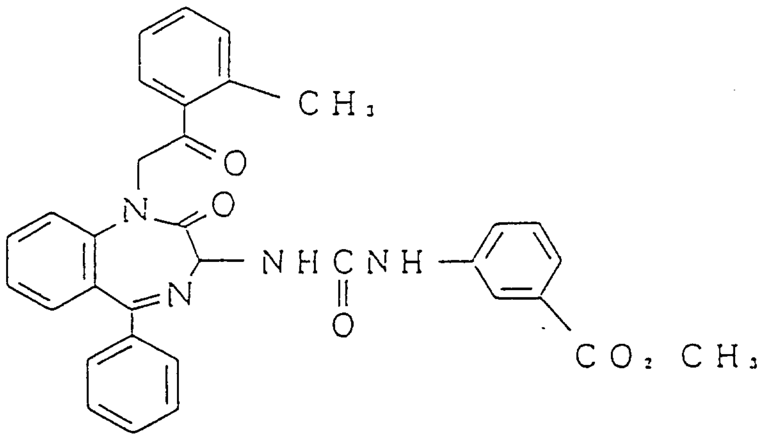

In the same manner as described in Example 3, except for using toluene in place of benzene described in Example 3, the compound of Example 8 was obtained.

Compound Produced:

Methyl 3-[3-[2,3-dihydro-1-(2'-methylphenacyl)-2-oxo-5-phenyl-1H-1,4-benzodiazepin-3-yl]ureido]-benzoate Starting Compound: Methyl 3-amino-1,3-dihydro-1-(2'-methylphenacyl)-5-phenyl-2H-1,4-benzodiazepin-2-one and methyl isophthalate

MP: 221-224 °C

| Elementary Analysis (for $C_{33}H_{28}N_4O_5$) | | | |
|---|---|---|---|
| | C (%) | H (%) | N (%) |
| Calc'd values: | 70.70 | 5.03 | 9.99 |
| Found values: | 70.60 | 4.99 | 9.94 |

MS FAB, Pos. (m/z): 561 ($M^+$ + 1)

EXAMPLE 9

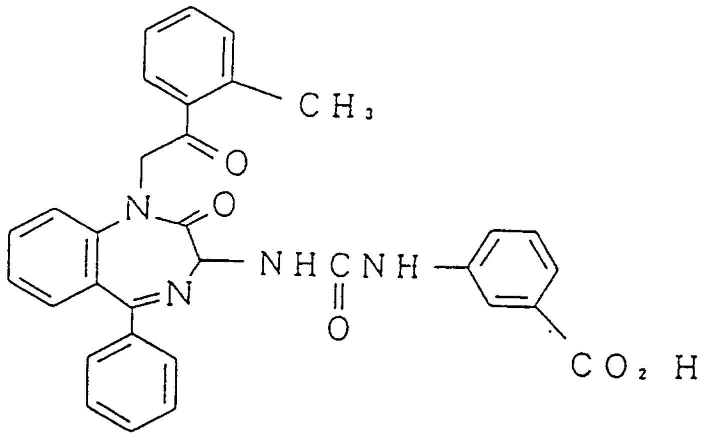

5.7 ml of a 1N aqueous sodium hydroxide solution was added to 32 ml of a methanol suspension of 1.07 g of methyl 3-[3-[2,3-dihydro-1-(2'-methylphenacyl)-2-oxo-5-phenyl-1H-1,4-benzodiazepin-3-yl]ureido]-benzoate (Compound of Example 8), and the mixture was stirred at 50 °C for 6 hours and then at room temperature for 18 hours.

5.7 ml of 1N hydrochloric acid and water were added to the reaction solution, and the precipitated insoluble material was separated by filtration. This product was subjected to silica gel column chromatography, and the column was eluted successively with acetone-chloroform (1:1), acetone, and methanol-chloroform (7:93). The fraction eluted with a mixture of methanol-chloroform was recrystallized from diethyl ether to obtain 0.73 g of 3-[3-[2,3-dihydro-1-(2'-methylphenacyl)-2-oxo-5-phenyl-1H-1,4-benzodiazepin-3-yl]-ureido]benzoic acid.

MP: 254-257 °C (decomposition)

| Elementary Analysis (for $C_{32}H_{26}N_4O_5 \cdot 0.4H_2O$) | | | |
|---|---|---|---|
| | C (%) | H (%) | N (%) |
| Calc'd values: | 69.40 | 4.88 | 10.12 |
| Found values: | 69.37 | 4.85 | 10.11 |

MS FAB, Pos. (m/z): 547 ($M^+$ + 1)

EXAMPLE 10

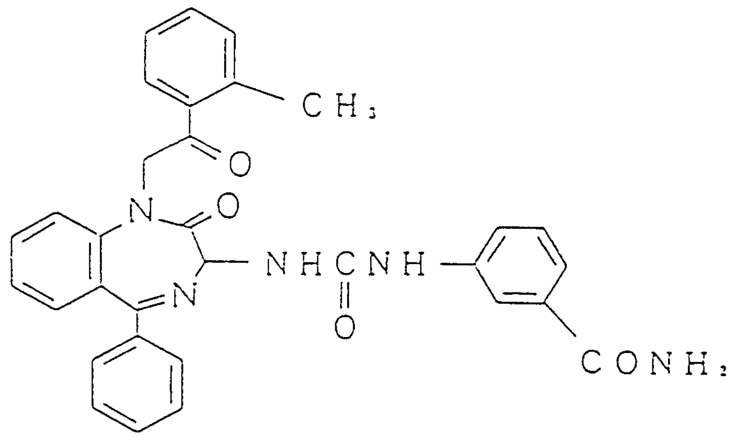

62 mg of N-methylmorpholine and 74 mg of isobutyl chloroformate were added to 4 ml of a tetrahydrofuran suspension of 300 mg of 3-[3-[2,3-dihydro-1-(2'-methylphenacyl)-2-oxo-5-phenyl-1H-1,4-benzodiazepin-3-yl]ureido]benzoic acid (Compound of Example 9) and, after stirring for 15 minutes at room temperature, 0.3 ml of concentrated aqueous ammonia was added thereto, followed by stirring for 14 hours.

The precipitated crystals were separated by filtration, and washed successively with 8 ml of methanol-water (1:1) and 4 ml of methanol to obtain 86 mg of 3-[3-[2,3-dihydro-1-(2'-methylphenacyl)-2-oxo-5-phenyl-1H-1,4-benzodiazepin-3-yl]ureido]benzamide.

MP: 271-275 °C (decomposition)

| Elementary Analysis (for $C_{32}H_{27}N_5O_4 \cdot 1.25H_2O$) | | | |
|---|---|---|---|
| | C (%) | H (%) | N (%) |
| Calc'd values: | 67.85 | 5.23 | 12.33 |
| Found values: | 67.85 | 4.83 | 12.08 |

MS FAB, Pos. (m/z): 546 ($M^+$ + 1)

EXAMPLE 11

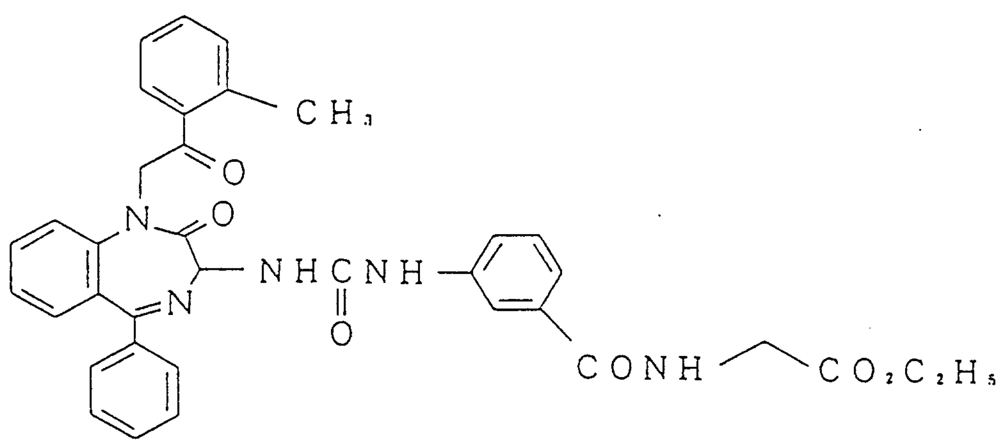

45 mg of triethylamine and 55 mg of isobutyl chloroformate were added to 3 ml of a tetrahydrofuran suspension of 220 mg of 3-[3-[2,3-dihydro-1-(2'-methylphenacyl)-2-oxo-5-phenyl-1H-1,4-benzodiazepin-3-yl]ureido]benzoic acid (Compound of Example 9) and, after stirring for 20 minutes at room temperature, 1

ml of a tetrahydrofuran solution of 84 mg of ethyl glycine hydrochloride and 61 mg of triethylamine was added thereto, followed by stirring for 3 hours. Water was added to the reaction solution, and the mixture was extracted twice with 20 ml of chloroform.

The organic layers were combined, washed successively with water and a saturated aqueous sodium chloride solution and dried over anhydrous sodium sulfate. The residue obtained by distilling off the solvent was subjected to silica gel column chromatography, and the fraction eluted with a mixture of ethyl acetate-n-hexane (3:2) was recrystallized from ethyl acetate-n-hexane to obtain 139 mg of N-[3-[3-[2,3-dihydro-1-(2'-methylphenacyl)-2-oxo-5-phenyl-1H-1,4-benzodiazepin-3-yl]ureido]benzoyl]glycine ethyl ester.

MS FAB, Pos. (m/z): 632 ($M^+$ + 1)

NMR ($CDCl_3$, TMS Internal Standard)

δ: 1.24 (3H, t), 2.38 (3H, s), 4.06-4.22 (4H, m), 5.13, 5.36 (each 1H, each d), 5.70 (1H, d), 7.03-7.66 (20H, m)

EXAMPLE 12

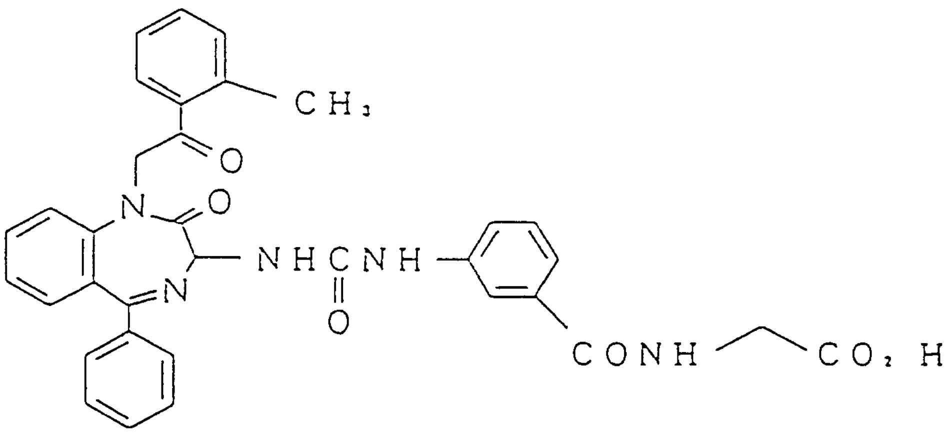

121 mg of N-[3-[3-[2,3-dihydro-1-(2'-methylphenacyl)-2-oxo-5-phenyl-1H-1,4-benzodiazepin-3-yl]ureido]-benzoyl]glycine ethyl ester was suspended in 4 ml of ethanol, and 0.57 ml of a 1N aqueous sodium hydroxide solution was added thereto, followed by stirring at room temperature for 2 hours. 0.57 ml of 1N hydrochloric acid and water were added to the reaction solution, and the precipitated crystals were separated by filtration and washed with water to obtain 104 mg of N-[3-[3-[2,3-dihydro-1-(2'-methylphenacyl)-2-oxo-5-phenyl-1H-1,4-benzodiazepin-3-yl]ureido]benzoyl]glycine.

MP: 169-174 °C

| Elementary Analysis (for $C_{34}H_{29}N_5O_6 \cdot 1.5H_2O$) | | | |
|---|---|---|---|
| | C (%) | H (%) | N (%) |
| Calc'd values: | 64.75 | 5.11 | 11.10 |
| Found values: | 64.89 | 4.96 | 10.97 |

MS FAB, Pos. (m/z): 604 ($M^+$ + 1)

EXAMPLE 13

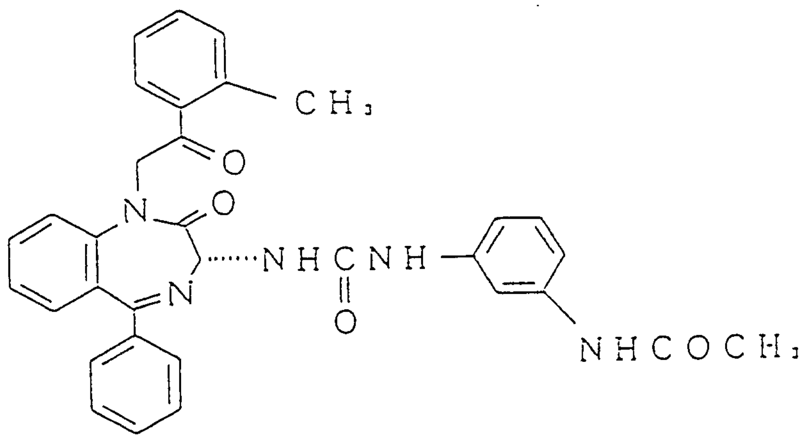

In the same manner as described in Example 3, except for using toluene-N,N-dimethylformamide in place of benzene described in Example 3, the compound of Example 13 was obtained.

Compound Produced:

(R)-1-(3-Acetylaminophenyl)-3-[2,3-dihydro-1-(2'-methylphenacyl)-2-oxo-5-phenyl-1H-1,4-benzodiazepin-3-yl]urea Starting Compound: (R)-3-Amino-1,3-dihydro-1-(2'-methylphenacyl)-5-phenyl-2H-1,4-benzodiazepin-2-one (S)-mandelate and 3-acetylaminobenzoic acid

Specific Rotation $[\alpha]_D^{20}$ = +121.1° (c=0.28, $CHCl_3$)

MP: 170-173°C

| Elementary Analysis (for $C_{33}H_{29}N_5O_4 \cdot 0.6H_2O$) | | | |
|---|---|---|---|
| | C (%) | H (%) | N (%) |
| Calc'd values: | 69.48 | 5.34 | 12.28 |
| Found values: | 69.56 | 5.39 | 12.00 |

MS FAB, Pos. (m/z): 560 ($M^+$ + 1)

EXAMPLE 14

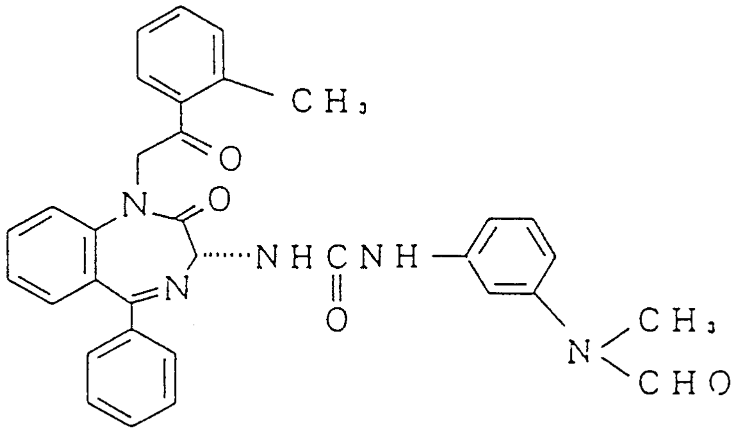

In the same manner as described in Example 3, except for using toluene in place of benzene described in Example 3, the compound of Example 14 was obtained.

Compound Produced:

(R)-1-[2,3-Dihydro-1-(2'-methylphenacyl)-2-oxo-5-phenyl-1H-1,4-benzodiazepin-3-yl]-3-[3-(N-formyl-N-methylamino)phenyl]urea
Starting Compound: (R)-3-Amino-1,3-dihydro-1-(2'-methylphenacyl)-5-phenyl-2H-1,4-benzodiazepin-2-one (S)-mandelate and 3-(N-formyl-N-methylamino)benzoic acid
Specific Rotation $[\alpha]_D^{20}$ = +123.4° (c=0.52, $CH_2Cl_2$)
MP: 125°C (decomposition)

| Elementary Analysis (for $C_{33}H_{29}N_5O_4 \cdot 0.3H_2O$) | | | |
|---|---|---|---|
| | C (%) | H (%) | N (%) |
| Calc'd values: | 70.15 | 5.28 | 12.39 |
| Found values: | 70.15 | 5.32 | 12.27 |

MS FAB, Pos. (m/z): 560 ($M^+$ + 1)

EXAMPLE 15

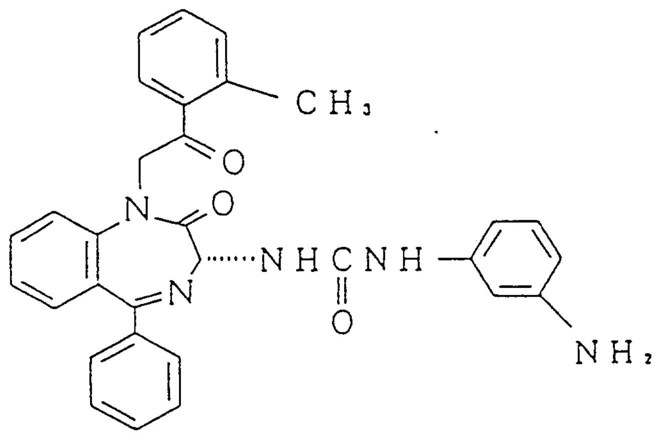

2 ml of 4N hydrochloric acid was added to 5 ml of an acetone solution of 350 mg of (R)-1-[2,3-dihydro-1-(2'-methylphenacyl)-2-oxo-5-phenyl-1H-1,4-benzodiazepin-3-yl]-3-(formylaminophenyl)urea (Compound of Example 6), followed by stirring at room temperature for 6 hours. Acetone was distilled off, and 10 ml of dichloromethane and 5 ml of a saturated aqueous sodium bicarbonate solution were added to the residue. After stirring, the organic layer was separated and dried over anhydrous magnesium sulfate.

The residue obtained by distilling off the solvent was subjected to silica gel column chromatography, and the fraction eluted with a mixture of ethyl acetate-n-hexane (2:1) was recrystallized from diethyl ether-dichloromethane to obtain 220 mg of (R)-1-(3-aminophenyl)-3-[2,3-dihydro-1-(2'-methylphenacyl)-2-oxo-5-phenyl-1H-1,4-benzodiazepin-3-yl]urea.

Specific Rotation $[\alpha]_D^{20}$ = +111.0° (c=0.39, $CHCl_3$)

MP: 165-168°C

| Elementary Analysis (for $C_{32}H_{27}N_5O_3 \cdot 0.55H_2O$) | | | |
|---|---|---|---|
| | C (%) | H (%) | N (%) |
| Calc'd values: | 71.24 | 5.25 | 12.98 |
| Found values: | 70.94 | 5.40 | 13.32 |

MS FAB, Pos. (m/z): 518 ($M^+$ + 1)

EXAMPLE 16

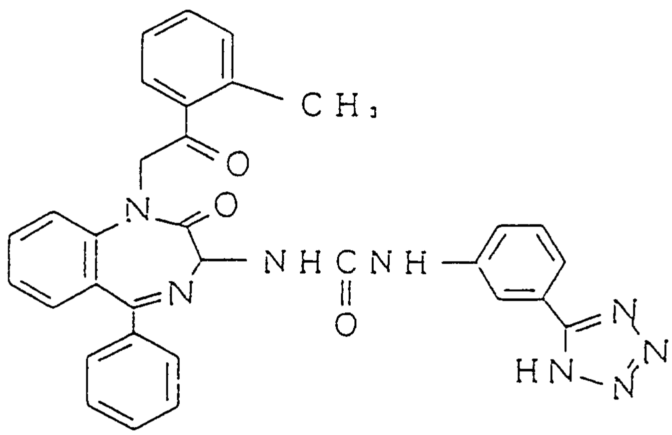

In the same manner as described in Example 3, except for using toluene-N,N-dimethylformamide in place of benzene described in Example 3, the compound of Example 16 was obtained.

Compound Produced:

1-[2,3-Dihydro-1-(2'-methylphenacyl)-2-oxo-5-phenyl-1H-1,4-benzodiazepin-3-yl]-3-[3-(5-tetrazolyl)-phenyl]urea Starting Compound: 3-Amino-1,3-dihydro-1-(2'-methylphenacyl)-5-phenyl-2H-1,4-benzodiazepin-2-one and 3-(5-tetrazolyl)benzoic acid

MP: 177-180 °C

| Elementary Analysis (for $C_{32}H_{26}N_8O_3 \cdot 1.15CHCl_3$) | | | |
|---|---|---|---|
| | C (%) | H (%) | N (%) |
| Calc'd values: | 63.33 | 4.72 | 17.82 |
| Found values: | 63.24 | 4.67 | 17.55 |

MS FAB, Pos. (m/z): 571 ($M^+$ + 1)

EXAMPLE 17

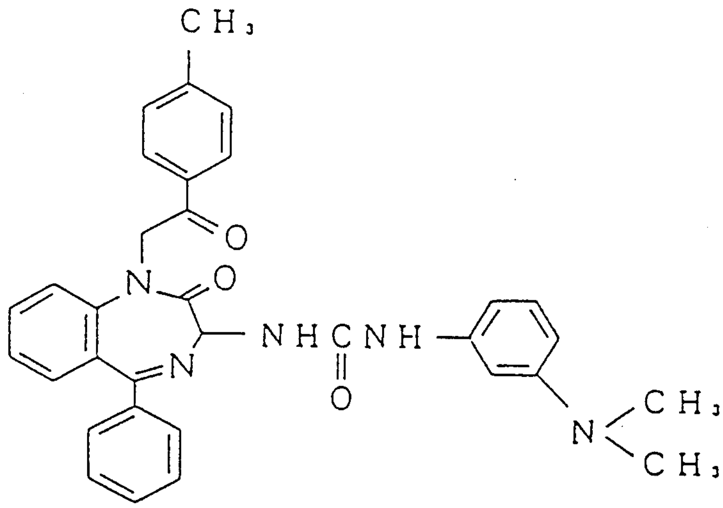

In the same manner as described in Example 3, except for using toluene in place of benzene described in Example 3, the compound of Example 17 was obtained.

Compound Produced:

1-[2,3-Dihydro-1-(4'-methylphenacyl)-2-oxo-5-phenyl-1H-1,4-benzodiazepin-3-yl]-3-(3-dimethylaminophenyl)urea Starting Compound: 1,3-Dihydro-1-(4'-methylphenacyl)-5-phenyl-2H-1,4-benzodiazepin-2-one and 3-dimethylaminobenzoic acid

MP: 180-183 °C

| Elementary Analysis (for $C_{33}H_{31}N_5O_3$) | | | |
|---|---|---|---|
| | C (%) | H (%) | N (%) |
| Calc'd values: | 72.64 | 5.73 | 12.84 |
| Found values: | 72.44 | 5.55 | 12.72 |

MS FAB, Pos. (m/z): 546 ($M^+$ + 1)

EXAMPLE 18

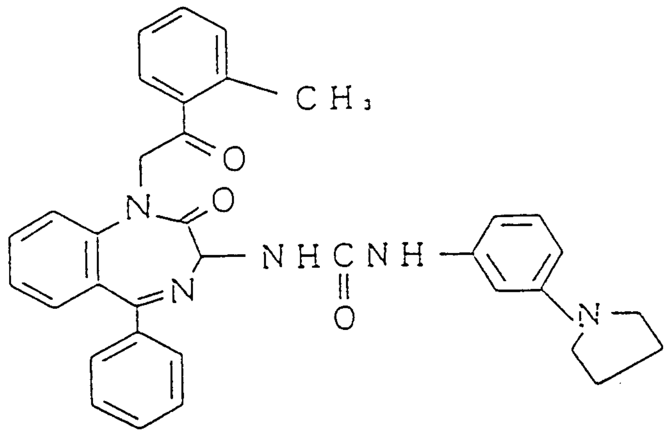

In the same manner as described in Example 3, except for using toluene in place of benzene described in Example 3, the compound of Example 18 was obtained.

Compound Produced:

1-[2,3-Dihydro-1-(2'-methylphenacyl)-2-oxo-5-phenyl-1H-1,4-benzodiazepin-3-yl]-3-[3-(1-pyrrolidinyl)-phenyl]urea Starting Compound: 3-Amino-1,3-dihydro-1-(2'-methylphenacyl)-5-phenyl-2H-1,4-benzodiazepin-2-one and 3-(1-pyrrolidinyl)benzoic acid

MP: 210 °C (decomposition)

| Elementary Analysis (for $C_{35}H_{33}N_5O_3$) | | | |
|---|---|---|---|
| | C (%) | H (%) | N (%) |
| Calc'd values: | 73.54 | 5.82 | 12.25 |
| Found values: | 73.33 | 6.11 | 12.11 |

MS FAB, Pos. (m/z): 572 ($M^+$ + 1)

EXAMPLE 19

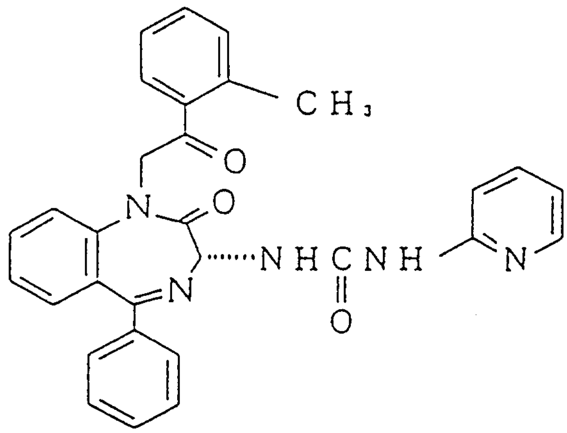

5 ml of a tetrahydrofuran solution of 148 mg of picolinic acid azide was added to 5 ml of a tetrahydrofuran solution of the free amine obtained by treating 150 mg of (R)-3-amino-1,3-dihydro-1-(2'-methylphenacyl)-5-phenyl-2H-1,4-benzodiazepin-2-one (S)-mandelate with dichloromethane and a 0.25N aqueous sodium hydroxide solution, and the mixture was stirred while heating under refluxing for 2 hours. After allowing the reaction solution to cool, the solvent was distilled off under reduced pressure, and the resulting residue was subjected to silica gel column chromatography. The fraction eluted with a mixture of ethyl acetate-n-hexane (1:1) was recrystallized from n-hexane to obtain 83 mg of (R)-1-[2,3-dihydro-1-(2'-methylphenacyl)-2-oxo-5-phenyl-1H-1,4-benzodiazepin-3-yl]-3-(2-pyridyl)urea.

MP: 138-140 °C

| Elementary Analysis (for $C_{30}H_{25}N_5O_3 \cdot 0.6H_2O$) | | | |
|---|---|---|---|
| | C (%) | H (%) | N (%) |
| Calc'd values: | 70.05 | 5.13 | 13.62 |
| Found values: | 70.14 | 4.92 | 13.33 |

MS FAB, Pos. (m/z): 504 ($M^+$ + 1)

EXAMPLE 20

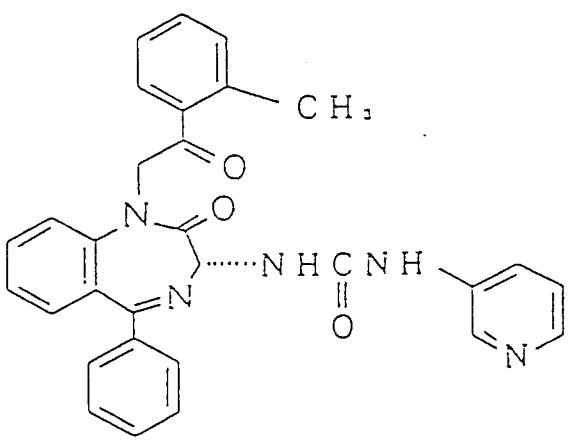

In the same manner as described in Example 19, the compound of Example 20 was obtained.

Compound Produced:

(R)-1-[2,3-Dihydro-1-(2'-methylphenacyl)-2-oxo-5-phenyl-1H-1,4-benzodiazepin-3-yl]-3-(3-pyridyl)urea
Starting Compound: (R)-3-Amino-1,3-dihydro-1-(2'-methylphenacyl)-5-phenyl-2H-1,4-benzodiazepin-2-one•-(S)-mandelate and nicotinic acid azide
MP: 243-245 °C

| Elementary Analysis (for $C_{30}H_{25}N_5O_3$) | | | |
|---|---|---|---|
| | C (%) | H (%) | N (%) |
| Calc'd values: | 71.56 | 5.00 | 13.91 |
| Found values: | 71.31 | 4.95 | 13.78 |

MS FAB, Pos. (m/z): 504 ($M^+$ + 1)

EXAMPLE 21

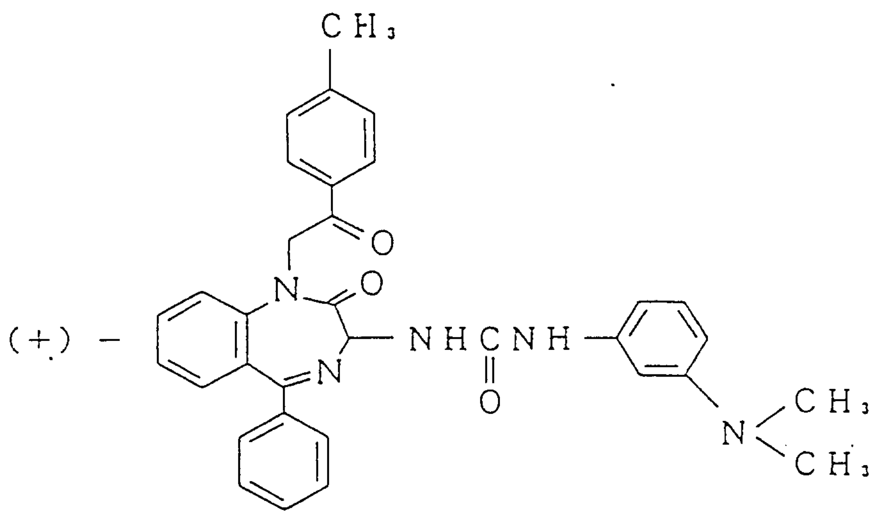

In the same manner as described in Example 3, except for using toluene in place of benzene, the compound of Example 21 was obtained.

Compound Produced:

(+)-1-[2,3-Dihydro-1-(4'-methylphenacyl)-2-oxo-5-phenyl-1H-1,4-benzodiazepin-3-yl]-3-(3-dimethylaminophenyl)urea
Starting Compound: (+)-3-Amino-1,3-dihydro-1-(4'-methylphenacyl)-5-phenyl-2H-1,4-benzodiazepin-2-one•2[(R)-mandelate]•monohydrate and 3-dimethylaminobenzoic acid
Specific Rotation $[\alpha]_D^{20}$ = +153.8° (c=1.01, $CH_2Cl_2$)
MP: 211-214 °C

| Elementary Analysis (for $C_{33}H_{31}N_5O_3$) | | | |
|---|---|---|---|
| | C (%) | H (%) | N (%) |
| Calc'd values: | 72.64 | 5.73 | 12.84 |
| Found values: | 72.59 | 5.72 | 12.92 |

MS FAB, Pos. (m/z): 546 ($M^+$ + 1)

EXAMPLE 22

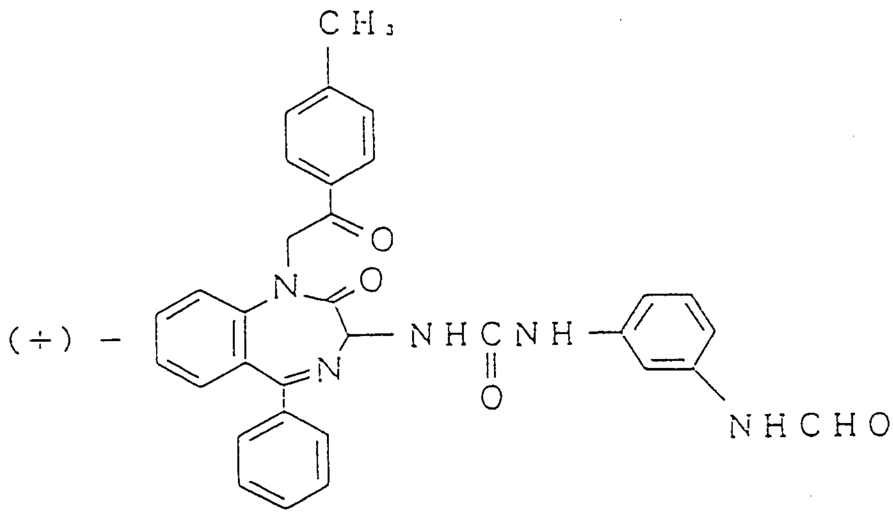

In the same manner as described in Example 3, except for using toluene-N,N-dimethylformamide in place of benzene, the compound of Example 22 was obtained.

Compound Produced:

(+)-1-[2,3-Dihydro-1-(4'-methylphenacyl)-2-oxo-5-phenyl-1H-1,4-benzodiazepin-3-yl]-3-(3-formylaminophenyl)urea Starting Compound: (+)-3-Amino-1,3-dihydro-1-(4'-methylphenacyl)-5-phenyl-2H-1,4-benzodiazepin-2-one•2[(R)-mandelate]•monohydrate and 3-formylaminobenzoic acid

Specific Rotation $[\alpha]_D^{20}$ = +151.0° (c=0.43, $CH_2Cl_2$)

MP: 169-172°C

| Elementary Analysis (for $C_{32}H_{27}N_5O_4 \cdot 0.6H_2O$) | | | |
|---|---|---|---|
| | C (%) | H (%) | N (%) |
| Calc'd values: | 69.08 | 5.11 | 12.59 |
| Found values: | 69.20 | 5.11 | 12.32 |

MS FAB, Pos. (m/z): 546 ($M^+$ + 1)

EXAMPLE 23

(+) -

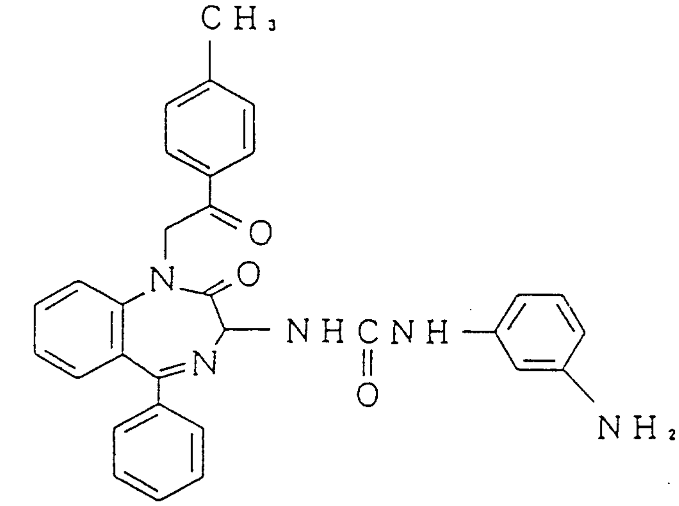

In the same manner as described in Example 15, the compound of Example 23 was obtained.

Compound Produced:

(+)-1-(3-Aminophenyl)-3-[2,3-dihydro-1-(4'-methylphenacyl)-2-oxo-5-phenyl-1H-1,4-benzodiazepin-3-yl]-urea Starting Compound: (+)-1-[2,3-Dihydro-1-(4'-methylphenacyl)-2-oxo-5-phenyl-2H-1,4-benzodiazepin-3-yl]-3-(3-formylaminophenyl)urea

Specific Rotation $[\alpha]_D^{20}$ = +148.6° (c=0.48, $CH_2Cl_2$)

MP: 159-163°C (decomposition)

| Elementary Analysis (for $C_{31}H_{27}N_5O_3 \cdot 0.4H_2O$) | | | |
|---|---|---|---|
| | C (%) | H (%) | N (%) |
| Calc'd values: | 70.95 | 5.34 | 13.35 |
| Found values: | 71.05 | 5.34 | 13.17 |

MS FAB, Pos. (m/z): 518 ($M^+$ + 1)

EXAMPLE 24

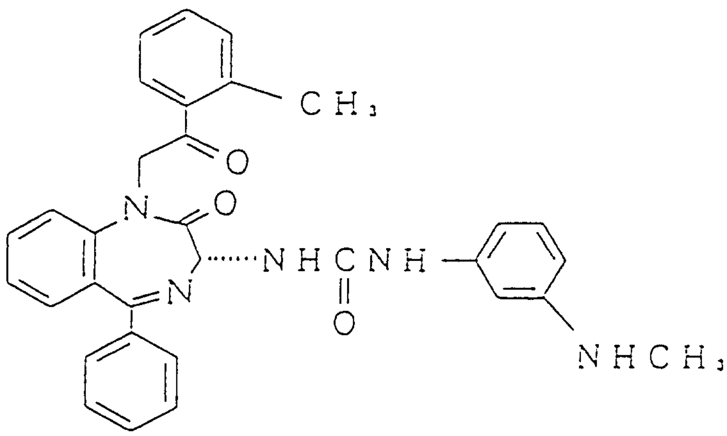

In the same manner as described in Example 15, the compound of Example 24 was obtained.

Compound Produced:

(R)-1-[2,3-Dihydro-1-(2'-methylphenacyl)-2-oxo-5-phenyl-1H-1,4-benzodiazepin-3-yl]-3-(3-methylaminophenyl)urea Starting Compound: (R)-1-[2,3-Dihydro-1-(2'-methylphenacyl)-2-oxo-5-phenyl-1H-1,4-benzodiazepin-3-yl]-3-[3-(N-formyl-N-methylamino)phenyl]urea

Specific Rotation $[\alpha]_D^{20}$ = +129.1° (c=0.17, $CHCl_3$)

MP: 151-153°C

| Elementary Analysis (for $C_{32}H_{29}N_5O_3$) | | | |
|---|---|---|---|
| | C (%) | H (%) | N (%) |
| Calc'd values: | 72.30 | 5.50 | 13.17 |
| Found values: | 72.61 | 6.09 | 12.32 |

MS FAB, Pos. (m/z): 532 ($M^+$ + 1)

EXAMPLE 25

(+) –

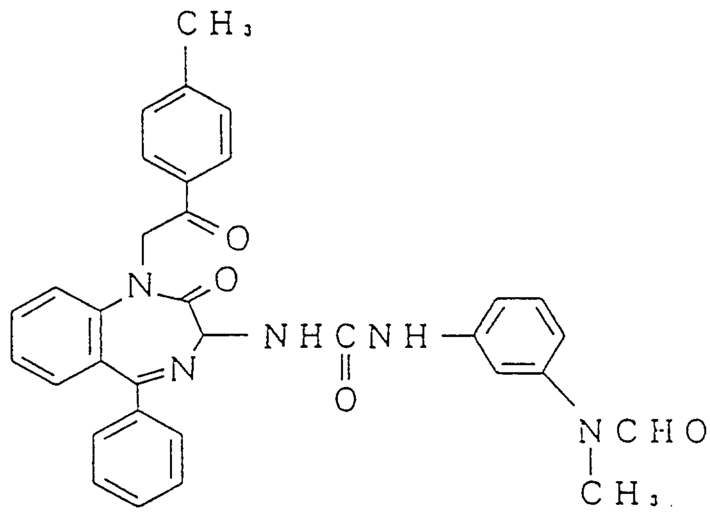

In the same manner as described in Example 3, except for using toluene in place of benzene, the compound of Example 25 was obtained.

Compound Produced:

(+)-1-[2,3-Dihydro-1-(4'-methylphenacyl)-2-oxo-5-phenyl-1H-1,4-benzodiazepin-3-yl]-3-[3-(N-formyl-N-methylamino)phenyl]urea

Starting Compound: (+)-3-Amino-1,3-dihydro-1-(4'-methylphenacyl)-5-phenyl-2H-1,4-benzodiazepin-2-one•2[(R)-mandelate]•monohydrate and 3-(N-formyl-N-methylamino)benzoic acid

Specific Rotation $[\alpha]_D^{20}$ = +136.8° (c=0.36, $CH_2Cl_2$)

MP: 152-155°C

| Elementary Analysis (for $C_{33}H_{29}N_5O_4 \cdot 0.5H_2O$) | | | |
|---|---|---|---|
| | C (%) | H (%) | N (%) |
| Calc'd values: | 69.70 | 5.32 | 12.32 |
| Found values: | 69.93 | 5.34 | 12.16 |

MS FAB, Pos. (m/z): 560 ($M^+$ + 1)

EXAMPLE 26

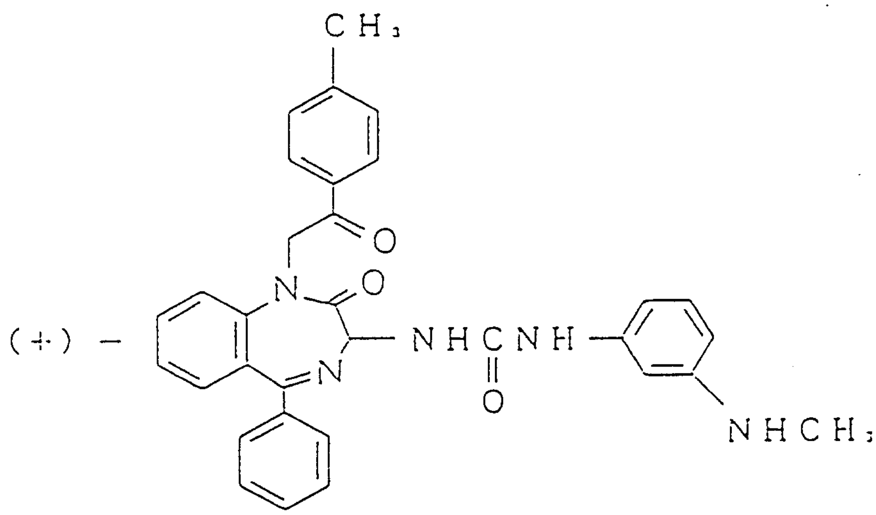

In the same manner as described in Example 15, the compound of Example 26 was obtained.

Compound Produced:

(+)-1-[2,3-Dihydro-1-(4'-methylphenacyl)-2-oxo-5-phenyl-1H-1,4-benzodiazepin-3-yl]-3-(3-methylaminophenyl)urea Starting Compound: (+)-1-[2,3-Dihydro-1-(4'-methylphenacyl)-2-oxo-5-phenyl-1H-1,4-benzodiazepin-3-yl]-3-[3-(N-formyl-N-methylamino)phenyl]urea

Specific Rotation $[\alpha]_D^{20}$ = +142.8° (c=0.29, $CHCl_3$)

MP: 148-151°C

| Elementary Analysis (for $C_{32}H_{29}N_5O_3 \cdot 0.8H_2O$) | | | |
|---|---|---|---|
| | C (%) | H (%) | N (%) |
| Calc'd values: | 70.39 | 5.65 | 12.83 |
| Found values: | 70.14 | 5.54 | 12.66 |

MS FAB, Pos. (m/z): 532 ($M^+$ + 1)

EXAMPLE 27

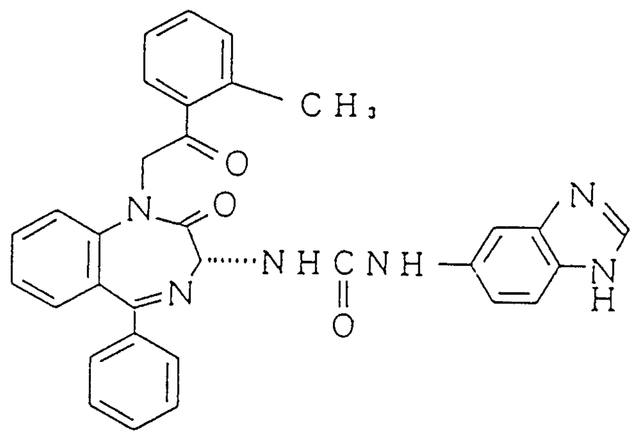

(a) 0.2 ml of triethylamine was added to 10 ml of a tetrahydrofuran solution of the free amine obtained by treating 750 mg of (R)-3-amino-1,3-dihydro-1-(2'-methylphenacyl)-5-phenyl-2H-1,4-benzodiazepin-2-one•(S)-mandelate with dichloromethane and a 0.25N aqueous sodium hydroxide solution, followed by stirring at 0°C for 30 minutes. 280 mg of 4-nitrophenyl chloroformate was added to the reaction solution, and the mixture was stirred at room temperature for 2 hours.

The solvent of the reaction solution was distilled off, and the residue was dissolved in 10 ml of N,N-dimethylformamide. Then, 522 mg of 5-amino-1-triphenylmethyl-1H-benzimidazole and 0.34 ml of triethylamine were added thereto, and the mixture was stirred at 45°C for 26 hours. The reaction solution was allowed to cool to room temperature, 10 ml of a saturated aqueous sodium bicarbonate solution and 30 ml of dichloromethane were added thereto. The organic layer was separated, washed successively with water and a saturated aqueous sodium chloride solution, and dried over anhydrous magnesium sulfate. The solvent was distilled off, the resulting residue was subjected to silica gel column chromatography, and 843 mg of (R)-1-[2,3-dihydro-1-(2'-methylphenacyl)-2-oxo-5-phenyl-1H-1,4-benzodiazepin-3-yl]-3-(1-triphenylmethyl-1H-benzimidazol-5-yl)urea was obtained from the fraction eluted with a mixture of hexane-ethyl acetate (1:4).

(b) 636 mg of (R)-1-[2,3-dihydro-1-(2'-methylphenacyl)-2-oxo-5-phenyl-1H-1,4-benzodiazepin-3-yl]-3-(1-triphenylmethyl-1H-benzimidazol-5-yl)urea was dissolved in 30 ml of acetone, and 3 ml of 4N hydrochloric acid was added thereto, followed by stirring at room temperature for 24 hours. The solvent was distilled off, and 20 ml of a saturated aqueous sodium bicarbonate solution and 30 ml of dichloromethane were added to the resulting residue. The organic layer was separated, washed successively with water and a saturated aqueous sodium chloride solution, and dried over anhydrous magnesium sulfate. The solvent was distilled off, the resulting residue was subjected to silica gel column chromatography, and the fraction eluted with a mixture of dichloromethane-methanol (30:1) was recrystallized from diethyl ether-dichloromethane to obtain 155 mg of (R)-1-(1H-benzimidazol-5-yl)-3-[2,3-dihydro-1-(2'-methylphenacyl)-2-oxo-5-phenyl-1H-1,4-benzodiazepin-3-yl]urea.

Specific Rotation $[\alpha]_D^{20}$ = +166.3° (c=0.69, $CHCl_3$)

MP: 182-185°C

| Elementary Analysis (for $C_{32}H_{26}N_6O_3 \cdot 0.8H_2O$) | | | |
|---|---|---|---|
| | C (%) | H (%) | N (%) |
| Calc'd values: | 69.00 | 4.99 | 15.09 |
| Found values: | 69.17 | 4.82 | 15.10 |

MS FAB, Pos. (m/z): 543 ($M^+$ + 1)

EXAMPLE 28

(+) -
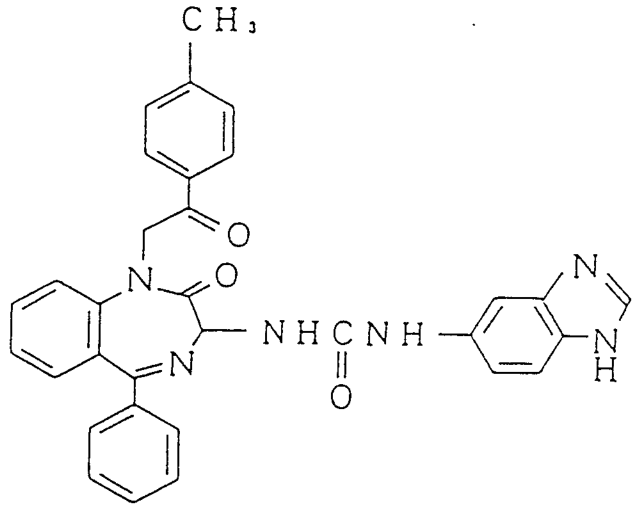

In the same manner as described in Example 27, the compound of Example 28 was obtained.

Compound Produced:

(+)-1-(1H-Benzimidazol-5-yl)-3-[2,3-dihydro-1-(4'-methylphenacyl)-2-oxo-5-phenyl-1H-1,4-benzodiazepin-3-yl]urea Starting Compound: (+)-3-Amino-1,3-dihydro-1-(4'-methylphenacyl)-5-phenyl-2H-1,4-benzodiazepin-2-one•2[(R)-mandelate]•monohydrate

Specific Rotation $[\alpha]_D^{20}$ = +123.0° (c=0.33, DMF)

MP: 203-206°C

| Elementary Analysis (for $C_{32}H_{26}N_6O_3.0.3H_2O$) | | | |
|---|---|---|---|
| | C (%) | H (%) | N (%) |
| Calc'd values: | 70.14 | 4.89 | 15.34 |
| Found values: | 69.80 | 4.84 | 15.35 |

MS FAB, Pos. (m/z): 543 ($M^+$ + 1)

EXAMPLE 29

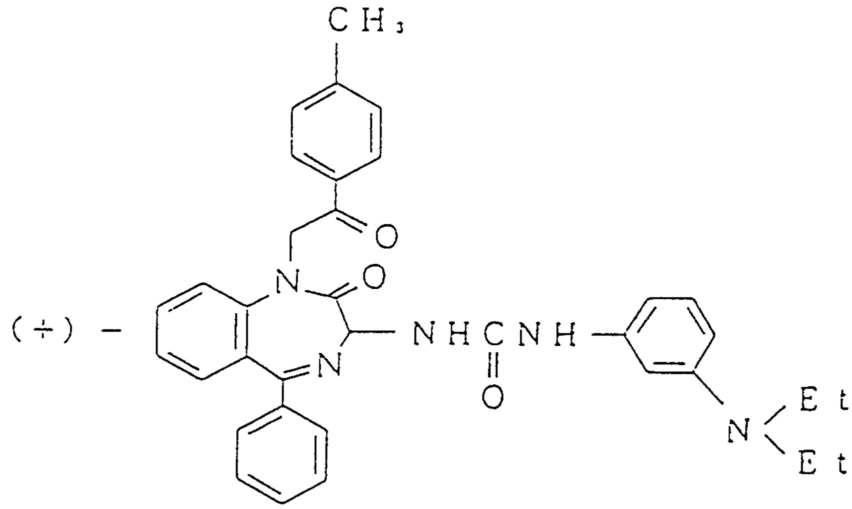

(a) 1.5 ml of an acetone solution of 102 mg of triethylamine and 0.5 ml of an acetone solution of 119 mg of ethyl chloroformate were successively added to a mixed solution of 169 mg of 3-diethylaminobenzoic acid and 2.5 ml of acetone under ice cooling, followed by stirring for 30 minutes. Then, 0.5 ml of an aqueous solution of 85 mg of sodium azide was added thereto, and the mixture was stirred at that temperature for one hour. Water was added to the reaction solution which was then extracted with 25 ml of toluene. The extract was washed with water and a saturated aqueous sodium chloride solution, and dried over anhydrous magnesium sulfate. The filtrate was heated for 45 minutes under refluxing and, after cooling, toluene was added thereto to make the total volume 35 ml. The resulting solution contained 4.75 mg of 3-diethylaminophenyl isocyanate per ml of the solution.

(b) 21 ml of a toluene solution of 3-diethylaminophenyl isocyanate obtained in (a) above was added to 2.5 ml of a toluene solution of the free amine obtained by treating 304 mg of (+)-3-amino-1,3-dihydro-1-(4'-methylphenacyl)-5-phenyl-2H-1,4-benzodiazepin-2-one•2[(R)-mandelate]•monohydrate with dichloromethane and a 0.25N aqueous sodium hydroxide, followed by stirring at room temperature for one hour. The precipitated crystals were separated by filtration and washed with toluene to obtain 204 mg of (+)-1-(3-diethylaminophenyl)-3-[2,3-dihydro-1-(4'-methylphenacyl)-2-oxo-5-phenyl-1H-1,4-benzodiazepin-3-yl]urea.

Specific Rotation $[\alpha]_D^{20}$ = +148.4° (c = 1.03, $CH_2Cl_2$)

MP: 235-239 °C

| Elementary Analysis (for $C_{35}H_{35}N_5O_3$) | | | |
|---|---|---|---|
| | C (%) | H (%) | N (%) |
| Calc'd values: | 73.28 | 6.15 | 12.21 |
| Found values: | 73.29 | 6.12 | 12.24 |

MS FAB, Pos. (m/z): 574 ($M^+$ + 1)

EXAMPLE 30

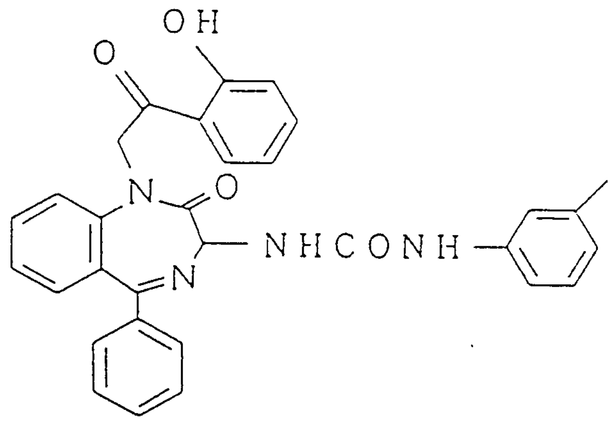

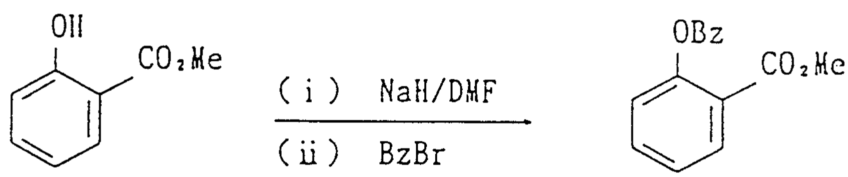


(wherein Bz represents a benzyl group, hereinafter the same)

Methyl salicylate (15.22 g, 100 mmol) was placed in dimethylformamide (250 ml) at 0 ° C and treated by adding sodium hydride (3 g, 1 equivalent, 80% oil preparation) in small portions over a period of 20 minutes. The mixture was stirred at 0 ° C for 20 minutes, and then treated with benzyl bromide (13.1 ml, 110 mmol). The mixture was stirred at room temperature for 18 hours, and distributed between ethyl acetate and 1M hydrochloric acid. The organic layer was washed with an aqueous sodium chloride solution, filtered and distilled off. The residue was purified by flash chromatography (eluent, 10% ethyl acetate-hexane) to obtain methyl 2-benzyloxybenzoate as a colorless oily substance (19.10 g, 79%).

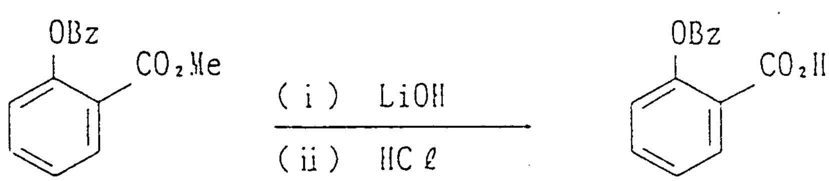


Methyl 2-benzyloxybenzoate (19.1 g, 79 mmol) was placed in dioxane/water and treated with lithium hydroxide monohydrate (5.0 g, 1.5 equivalent) at room temperature for 18 hours. The mixture was concentrated and distributed between ethyl acetate and 1M hydrochloric acid. The organic layer was washed with an aqueous sodium chloride solution, filtered and distilled off to obtain 2-benzyloxybenzoic acid as a colorless oily substance (18.3 g, 100%).

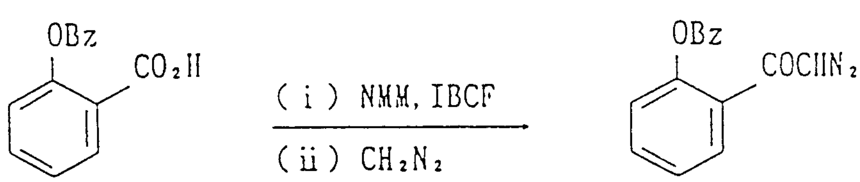

2-Benzyloxybenzoic acid (4.4 g, 19.3 mmol) was placed in tetrahydrofuran (50 ml) at -20 °C, and treated with N-methylmorpholine (2.1 ml, 20 mmol) and isobutyl chloroformate (2.4 mol, 20 mmol), followed by stirring at a temperature between -20 °C to -15 °C for 30 minutes. Then, the resulting mixture was poured into an ethanolic solution of diazomethane (generated from Diazald (a registered trademark) 10.75 ml, 50 mmol). The mixture was stirred at room temperature for 90 minutes, and acetic acid was carefully added thereto to decompose any excess of diazomethane. The resulting solution was washed with a 5% potassium bicarbonate solution and an aqueous sodium chloride solution, and distilled off. The residue was purified by flash chromatography (eluent, 15% ethyl acetate-hexane) to obtain 2'-benzyloxy-2-diazoacetophenone as a yellow oily substance (900 mg, 19%).

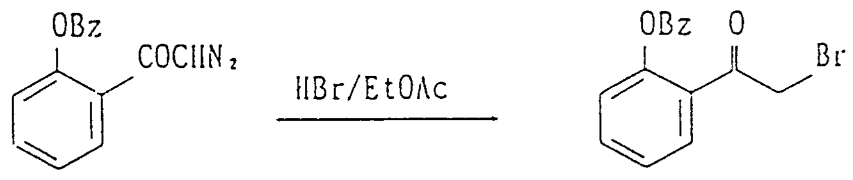


2'-Bezyloxy-2-diazoacetophenone (900 mg, 3.6 mmol) was placed in ethyl acetate (50 ml) at -20 °C, and the mixture was treated by adding dropwise thereto a 3M hydrogen bromide-ethyl acetate solution (1.5 ml, 45 mmol). After 10 minutes, the mixture was rendered basic with a 5% aqueous potassium bicarbonate, warmed to room temperature, and the product was extracted with ethyl acetate. The organic layer was washed with an aqueous sodium chloride solution, filtered and distilled. The residue was purified by flash chromatography (eluent: 15% ethyl acetate-hexane) to obtain 2'-benzyloxy-2-bromoacetophenoneas a colorless oily substance (280 mg, 26%).

NMR ($CDCl_3$)

δ: 7.70-6.95 (9H, m), 5.05 (2H, s), 4.50 (2H, s)

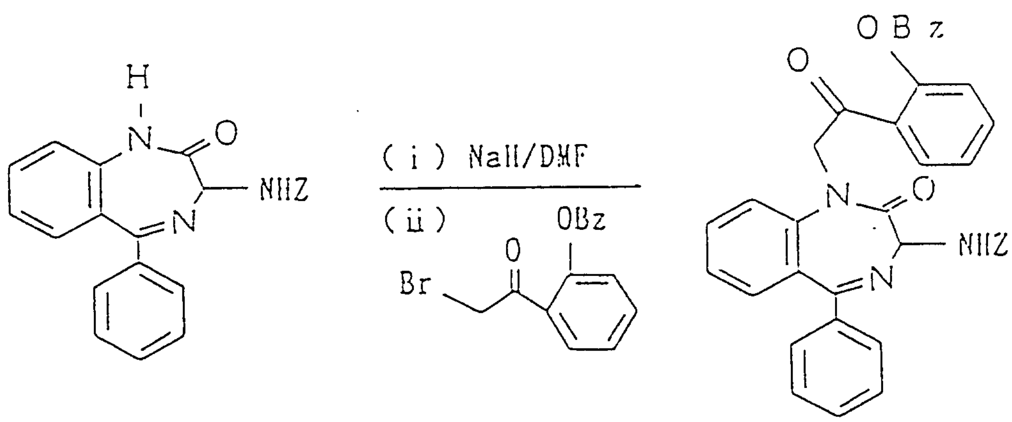


3-Benzyloxycarbonylamino-1,3-dihydro-5-phenyl-2H-1,4-benzodiazepin-2-one hydrate (322 mg, 0.8 mmol) was azeotropically boiled with dimethylformamide, and sodium hydride (34 mg, 1.4 equivalent, 80% oil preparation) was added to the stirred solution at 0 °C. After stirring at 0 °C for 45 minutes, 2'-benzyloxy-2-bromoacetophenone (280 mg, 0.92 mmol) was added thereto. After stirring at room temperature for 90 minutes, the mixture was distributed between ethyl acetate and 1M hydrochloric acid, and the organic layer was washed with an aqueous sodium chloride solution, filtered and distilled. The residue was purified by flash chromatography (eluent: 40% ethyl acetate-hexane) to obtain 3-benzyloxycarbonylamino-1-(2'-benzyloxyphenacyl)-1,3-dihydro-5-phenyl-2H-1,4-benzodiazepin-2-one (330 mg, 68%).

NMR ($CDCl_3$)

δ: 7.60 (1H, d, J=8Hz), 7.40-6.90 (21H, m), 6.50 (1H, d, J=8Hz), 5.30 (1H, d, J=8Hz), 5.10-4.90 (6H, m)

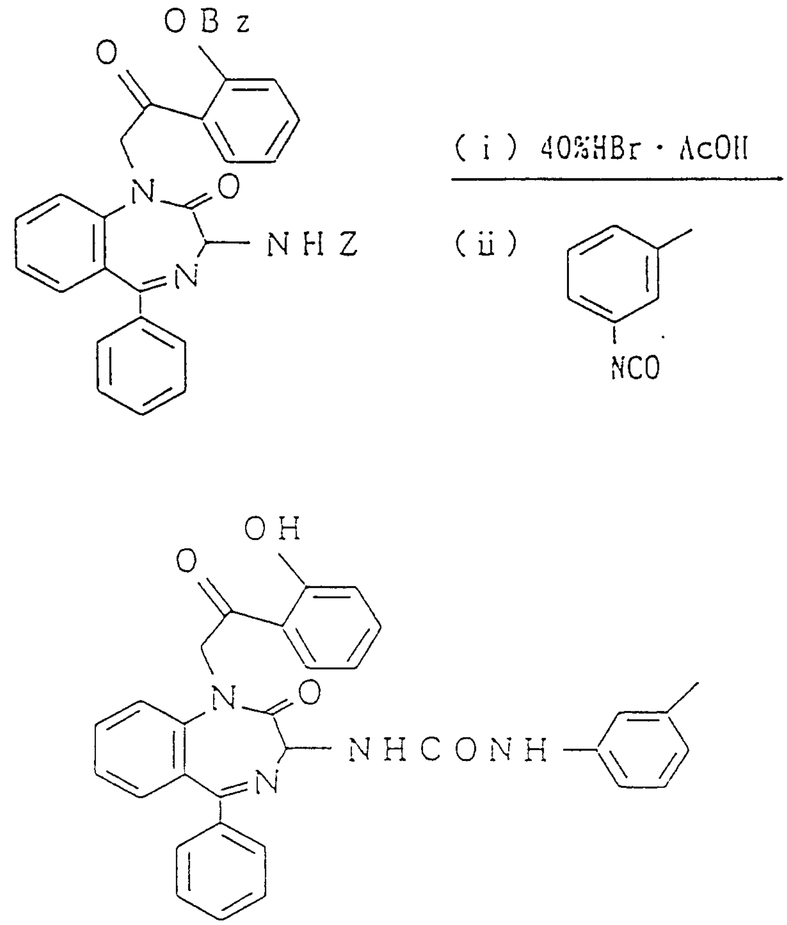


3-Benzyloxycarbonylamino-1-(2'-benzyloxyphenacyl)-1,3-dihydro-5-phenyl-2H-1,4-benzoadiazepin-2-one (330 mg, 0.54 mmol) was treated with a 40% hydrogen bromide-acetic acid solution (20 ml) at room temperature for 2 hours. Then, the mixture was distilled and azeotropically boiled with toluene. The mixture was placed in dichloromethane (5 ml), and treated with m-tolyl isocyanate (85 $\mu$l, 0.65 mmol) at room temperature for one hour. The reaction mixture was concentrated, purified by silica gel flash chromatography (eluent: 40% ethyl acetate-hexane) and freeze-dried from acetonitrile/water to obtain 1-[2,3-dihydro-1-(2'-hydroxyphenacyl)-2-oxo-5-phenyl-1H-1,4-benzodiazepin-3-yl]-3-(3-tolyl)urea (41 mg, 15%)

[M+H] = 519.4

NMR ($CDCl_3$)

$\delta$: 12.5 (1H, s), 7.50-6.80 (19H, m), 5.80 (1H, d, J=8Hz), 5.35 (1H, d, J=17Hz), 5.20 (1H, d, J=17Hz), 2.05 (3H, s)

EXAMPLE 31

(a)

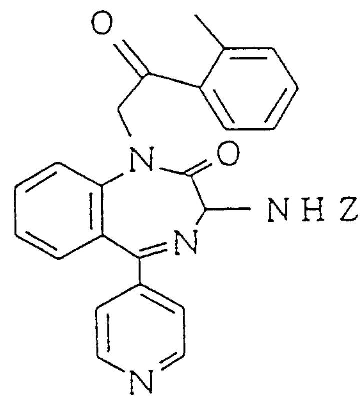

Sodium hydride (32 mg, 80% oil preparation) was added to a stirred dry dimethylformamide (6 ml) solution of 3-benzyloxycarbonylamino-1,3-dihydro-5-(4-pyridyl)-2H-1,4-benzodiazepin-2-one (Freidinger et al., European Patent No. 0 434 364 A2) (303 mg, 0.75 mmol) at 0°C. The resulting mixture was stirred at 0°C for 30 minutes, and 2-bromo-2'-methylacetophenone (220 mg, 1.04 mmol) was added thereto. The mixture was stirred at room temperature for 2 hours and then distributed between ethyl acetate and an aqueous sodium chloride solution. The organic layer was dried over magnesium sulfate and distilled off. The residue was purified by chromatography (eluent: 95% ethyl acetate-hexane) to obtain 3-benzyloxycarbonylamino-1,3-dihydro-1-(2'-methylphenacyl)-5-(4-pyridyl)-2H-1,4-benzodiazepin-2-one as a white solid (345 mg, 89%).

TLC (ethyl acetate) Rf = 0.4

$^1$H NMR ($CDCl_3$, 270MHz)

δ: 8.68 (d, 2H, J=7Hz), 7.6-7.0 (m, 15H), 6.73 (d, 1H, J=9Hz), 5.5 (d, 1H, J=9Hz), 5.37 (d, 1H, J=16.5Hz), 5.11 (s, 2H), 5.04 (d, 1H, J=16.5Hz), 2.2 (s, 3H) ppm

(b)

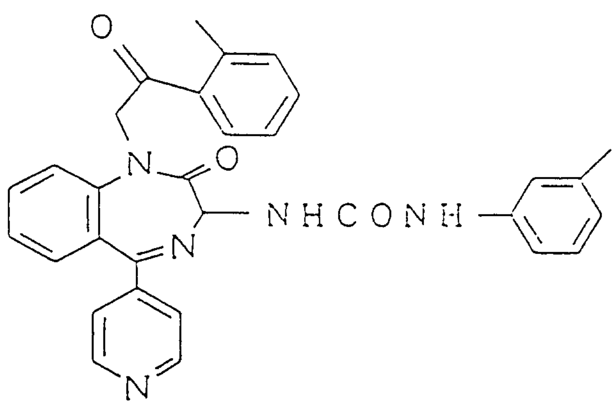

3-Benzyloxycarbonylamino-1,3-dihydro-1-(2'-methylphenacyl)-5-(4-pyridyl)-2H-1,4-benzodiazepin-2-one (340 mg, 0.656 mmol) was treated with a 40% hydrogen bromide-acetic acid solution (30 ml) at room temperature for 90 minutes. The resulting mixture was degassed and distilled off. The residue was distributed between ethyl acetate and 1M NaOH, and the organic layer was washed with an aqueous sodium chloride solution, filtered (Whatman IPS filter paper) and distilled off. The residue was placed in dichloromethane (5 ml) and treated with m-tolyl isocyanate (100 μl) at room temperature for 2 hours. The mixture was distilled off, and the residue was purified by chromatography (eluent, chloroform/methanol/acetic acid = 250/2/1 by volume) to obtain 1-[2,3-dihydro-1-(2'-methylphenacyl)-2-oxo-5-(4-pyridyl)-1H-1,4-benzodiazepin-3-yl]-3-(3-tolyl)urea as a white solid (96 mg, 28%).

TLC (ethyl acetate) Rf = 0.45

$^1$H NMR ($CDCl_3$, 270MHz)

δ: 8.62 (d, 2H, J=7Hz), 7.6-7.0 (m, 15H), 6.8 (d, 1H, J=9Hz), 5.76 (d, 1H, J=9Hz), 5.41 (d, 1H,

J = 18Hz), 4.97 (d, 1H, J = 18Hz), 2.31 (s, 3H), 2.22 (s, 3H) ppm

EXAMPLE 32

( a )

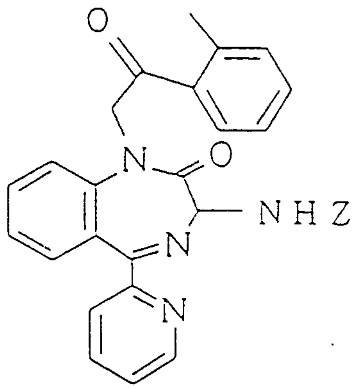

Sodium hydride (42 mg, 80% oil preparation) was added to a stirred dry dimethylformamide (8 ml) solution of 3-benzyloxycarbonylamino-1,3-dihydro-5-(2-pyridyl)-2H-1,4-benzodiazepin-2-one (Freidinger et al., European Patent No. 0 434 364 A2) (404 mg, 1 mmol) at 0 ° C. The resulting mixture was stirred at 0 ° C for 30 minutes, and 2-bromo-2'-methylacetophenone (275 mg, 1.3 mmol) was added thereto. The mixture was stirred at room temperature for one hour and then distributed between ethyl acetate and an aqueous sodium chloride solution. The organic layer was filtered (Whatman IPS filter paper) and distilled. The residue was purified by chromatography (eluent: 85% ethyl acetate-hexane) to obtain 3-benzyloxycarbonylamino-1,3-dihydro-1-(2'-methylphenacyl)-5-(2-pyridyl)-2H-1,4-benzodiazepin-2-one as a white solid (382 mg, 24%).

TLC (ethyl acetate) Rf = 0.55

$^1$H NMR ($CDCl_3$, 270MHz)

δ: 8.60 (d, 2H, J = 6Hz), 8.08 (d, J = 9Hz), 7.8-7.2 (m, 15H), 6.78 (d, 1H, J = 9Hz), 5.60 (d, 1H, J = 9Hz), 5.14 (d, 1H, J = 16Hz), 5.18 (s, 2H), 4.86 (d, 1H, J = 16Hz), 2.5 (s, 3H) ppm

( b )

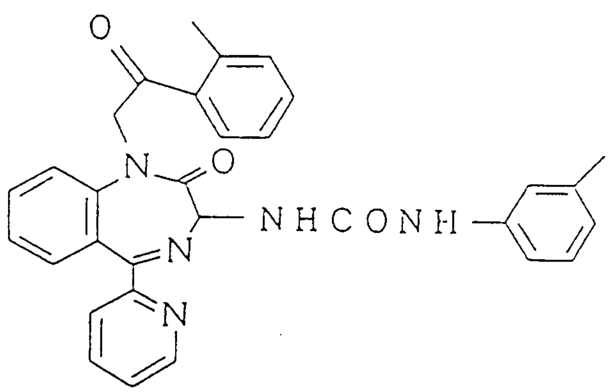

In acetic acid, 3-Benzyloxycarbonylamino-1,3-dihydro-1-(2'-methylphenacyl)-5-(2-pyridyl)-2H-1,4-benzodiazepin-2-one (375 mg, 0.724 mmol) was treated with a 40% hydrogen bromide-acetic acid solution in the same manner as described in Example 31. The resulting product was purified by chromatography (eluent: 75% ethyl acetate-hexane) and recrystallized from acetonitrile to obtain 1-[2,3-dihydro-1-(2'-methylphenacyl)-2-oxo-5-(2-pyridyl)-1H-1,4-benzodiazepin-3-yl]-3-(3-tolyl)urea as a white solid (94 mg, 25%).

TLC (ethyl acetate) Rf = 0.6

$^1$H NMR ($CDCl_3$, 270MHz)

δ: 8.60 (d, 1H, J = 6Hz), 7.95 (d, 1H, J = 8Hz), 7.7-7.0 (m, 14H), 6.80 (d, 1H, J = 9Hz), 5.78 (d, 1H, J = 9Hz), 5.15 (d, 1H, J = 17Hz), 5.01 (d, 1H, J = 17Hz), 2.46 (s, 3H), 2.22 (s, 3H) ppm

EXAMPLES 33 AND 34

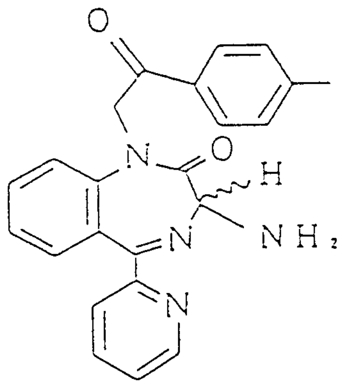 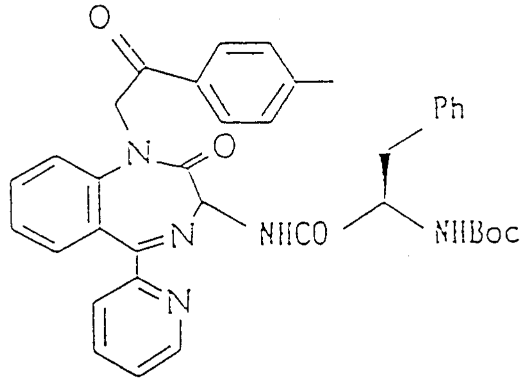

Bis(2-oxo-3-oxazolidinyl)-phosphine chloride (BOP-Cl, 0.87 g, 3.43 mmol) was added to a stirred dichloromethane (50 ml) solution of N-(t-butoxycarbonyl)-D-phenylanaline (0.84 g, 3.15 mmol) and diisopropylethylamine (0.65 ml, 3.72 mmol) cooled to -10 ° C. The resulting mixture was stirred at -20 ° C for 20 minutes, and then 3-amino-1,3-dihydro-1-(4'-methylphenacyl)-5-(2-pyridyl)-2H-1,4-benzodiazepin-2-one (1.1 g, 2.86 mmol) were added thereto. The mixture was stirred at room temperature overnight, then diluted with dichloromethane, washed successively with a saturated aqueous sodium bicarbonate solution and an aqueous sodium chloride solution, filtered (Whatman (a registered trademark) IPS layer separator) and distilled off under reduced pressure. The residue was purified by silica gel flash chromatography (eluent: ethyl acetate-hexane, 50:50 by volume) to obtain 1,1-dimethylethyl [(R)-2-[[2,3-dihydro-1-(4'-methylphenacyl)-2-oxo-5-(2-pyridyl)-1H-1,4-benzodiazepin-3-yl]amino]-2-oxo-1-(phenylmethyl)ethyl]carbamate (0.49 g, 27%).

Rf (ethyl acetate:hexane, 80:20 by volume): 0.40

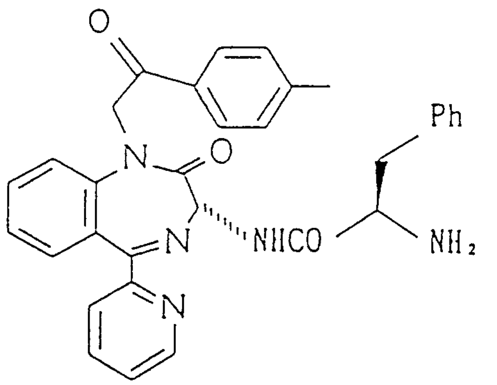 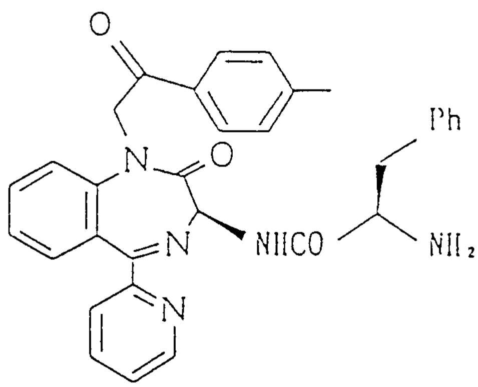

1,1-Dimethylethyl [(R)-2-[[2,3-dihydro-1-(4'-methylphenacyl)-2-oxo-5-(2-pyridyl)-1H-1,4-benzodiazepin-3-yl]amino]-2-oxo-1-(phenylmethyl)ethyl]carbamate (0.49 g, 0.776 mmol) was placed in a 4N hydrochloric acid-dioxane solution (50 ml), and the resulting solution was stirred at room temperature for 2 hours. The solvent was removed under reduced pressure, and the solution was finally azeotropically boiled with toluene. The residue was purified by silica gel flash chromatography (eluent: chloroform:methanol:acetic acid = 20:2:1 to 20:3:1.5 by volume) to obtain 2(R)-amino-N-[2,3-dihydro-1-(4'-methylphenacyl)-2-oxo-5-(2-pyridyl)-1H-1,4-benzodiazepin-3(R)-yl]benzenepropaneamide (0.161 g, 39%).

Rf (chloroform:methanol:acetic acid = 20:2:1 by volume): 0.38

$^1H$ NMR

$\delta$: 9.02 (1H, d, J = 8.25Hz), 8.63 (1H, dd, J = 4.95 and 0.99Hz), 8.12 (1H, d, J = 7.92), 7.83 (3H, m), 7.58-7.10 (12H, m), 5.77 (1H, d, J = 7.92), 5.44 (1H, d, J = 17.5Hz), 5.05 (1H, d, J = 17.5Hz), 3.75 (1H, dd, J = 10.0 and 3.70Hz), 2.71 (1H, dd, J = 13.9 and 10.0Hz), 2.39 (3H, s), 2.32 (2H, br, s).

Upon continuing elution from the column, 2(R)-amino-N-[2,3-dihydro-1-(4'-methylphenacyl)-2-oxo-5-(2-pyridyl)-1H-1,4-benzodiazepin-3(S)-yl]benzenepropaneamide could also be obtained (0.101 g, 24%).

Rf (chloroform:methanol:acetic acid = 20:2:1 by volume): 0.18

$^1H$ NMR

δ: 9.02 (1H, d, J = 8.24Hz), 8.63 (1H, dd, J = 4.62 and 0.99Hz), 8.15 (1H, d, J = 7.91), 7.83 (3H, m), 7.60-7.12 (12H, m), 5.79 (1H, d, J = 8.24), 5.44 (1H, d, J = 17.5Hz), 5.02 (1H, d, J = 17.5Hz), 3.71 (1H, dd, J = 9.89 and 3.95Hz), 3.35 (1H, dd, J = 13.9 and 3.95 Hz), 2.82 (1H, dd, J = 13.9 and 9.89Hz), 2.38 (3H, s)

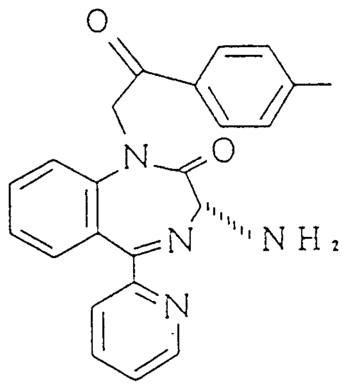

Phenyl isothiocyanate (43 µl, 0.363 mmol) was added to a stirred chloroform (5 ml) solution of 2(R)-amino-N-[2,3-dihydro-1-(4'-methylphenacyl-2-oxo-5-(2-pyridyl)-1H-1,4-benzodiazepin-3(R)-yl]-benzenepropaneamide (0.161 g, 0.303 mmol). The resulting mixture was heated for 5 hours under refluxing. The solvent was distilled off under reduced pressure, and the residue was purified by silica gel flash chromatography (eluent, ethyl acetate:hexane = 70:30 by volume) to obtain a thiourea. This thiourea was dissolved in trifluoroacetic acid (4 ml), and the solution was stirred at 50 °C for 4 hours. Trifluoroacetic acid was distilled off, and the residue was purified by column chromatography (eluent, chloroform:methanol:acetic acid = 20:2:1 by volume) to obtain (R)-3-amino-1,3-dihydro-1-(4'-methylphenacyl)-5-(2-pyridyl)-2H-1,4-benzodiazepin-2-one (93 mg, 80%).

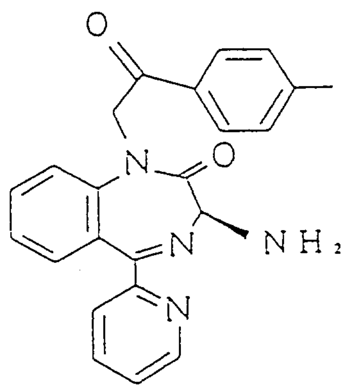

(S)-3-Amino-1,3-dihydro-1-(4'-methylphenacyl)-5-(2-pyridyl)-2H-1,4-benzodiazepin-2-one was prepared according to the above-described process using 2(R)-amino-N-[2,3-dihydro-1-(4'-methylphenacyl)-2-oxo-5-(2-pyridyl)-1H-1,4-benzodiazepin-3-(S)-yl]benenepropaneamide on a scale of 0.19 mmol. The product was isolated in a yield of 73% (53 mg) after silica gel flash chromatography (eluent, chloroform:methanol:acetic acid = 20:2:1 by volume).

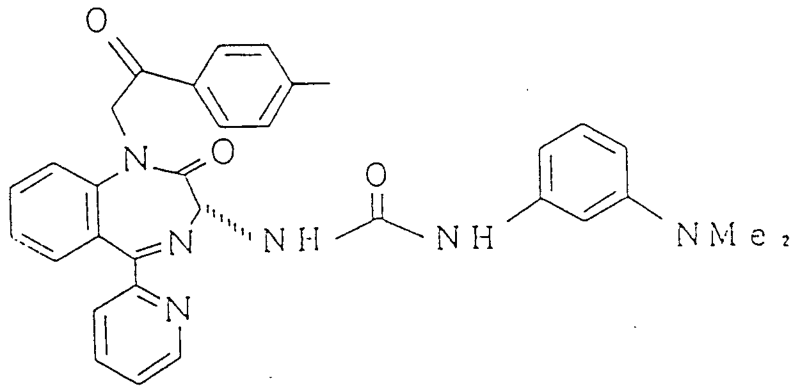

Diphenylphosphoryl azide (0.291 ml, 0.65 mmol) was added to a stirred benzene (4 ml) solution of 3-dimethylaminobenzoic acid (80 mg, 0.445 mmol) and triethylamine (90 μl, 0.65 mmol). The resulting mixture was heated for 4 hours under refluxing. The solvent was distilled off under reduced pressure, and the residue was dissolved in dichloromethane (1 ml), and a dichloromethane (1 ml) solution of (R)-3-amino-1,3-dihydro-1-(4'-methylphenacyl)-5-(2-pyridyl)-2H-1,4-benzodiazepin-2-one (43 mg, 0.112 mmol) was added to the above solution. The mixture was stirred at room temperature for 16 hours, and the solvent was distilled off under reduced pressure. The residue was purified by flash chromatography (eluent, ethyl acetate:hexane = 70:30 to 100:0 by volume) to obtain (R)-1-[2,3-dihydro-1-(4'-methylphenacyl)-2-oxo-5-(2-pyridyl)-1H-1,4-benzodiazepin-3-yl]-3-(3-dimethylaminophenyl)urea (34 mg, 56%).

Rf (chloroform:methanol:acetic acid = 20:2:1 by volume): 0.55

MS (FAB) m/e = 547.4 $[M+H]^+$

$^1H$ NMR

δ: 8.61 (1H, d, J=4.95Hz), 8.08 (1H, d, J=7.92Hz), 7.77 (3H, m), 7.52-7.03 (10H, m), 6.89 (1H, s), 6.53 (1H, m), 6.40 (1H, m), 5.80 (1H, d, J=7.92Hz), 5.30 (1H, d, J=17.5Hz), 5.04 (1H,d, J=17.5Hz), 2.88 (6H, s), 2.36 (3H, s)

$[\alpha]_D$ (c=0.34, chloroform): +105°

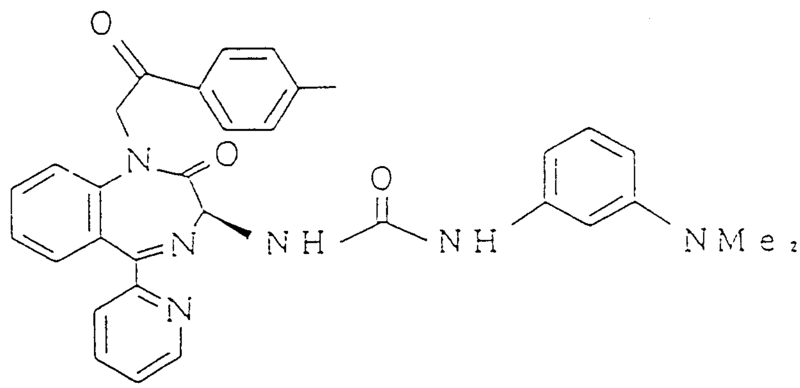

(S)-1-[2,3-dihydro-1-(4'-methylphenacyl)-2-oxo-5-(2-pyridyl)-1H-1,4-benzodiazepin-3-yl]-3-(3-dimethylaminophenyl)urea was prepared according to the above-described method using (S)-3-amino-1,3-dihydro-1-(4'-methylphenacyl)-5-(2-pyridyl)-2H-1,4-benzodiazepin-2-one on a scale of 0.14 mmol. The product was isolated in a yield of 28% (21 mg) after silica gel flash chromatography (eluent, ethyl acetate:hexane = 80:20 by volume).

Rf (chloroform:methanol:acetic acid = 20:2:1 by volume): 0.55

MS (FAB) m/e = 547.3 $[M+H]^+$

$^1H$ NMR

δ: 8.60 (1H, d, J=4.95Hz), 8.08 (1H, d, J=7.90Hz), 7.78 (3H, m), 7.50-7.03 (10H, m), 6.89 (1H, s), 6.53 (1H, m), 6.41 (1H, m), 5.80 (1H, d, J=7.90Hz), 5.32 (1H, d, J=17.5Hz), 5.04 (1H, d, J=17.5Hz), 2.88 (6H, s), 2.36 (3H, s)

$[\alpha]_D$ (c=0.34, chloroform): -124°

EXAMPLE 35

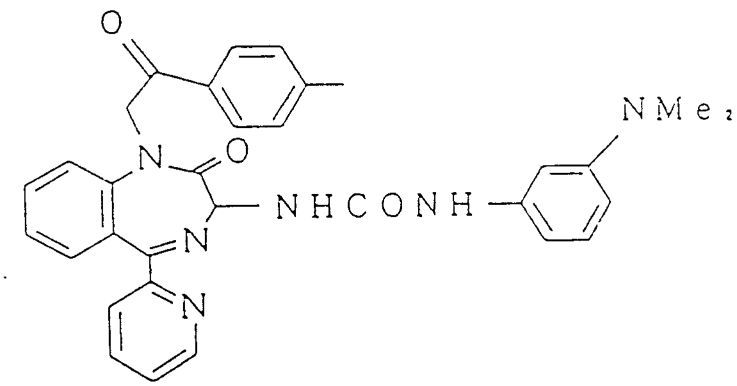

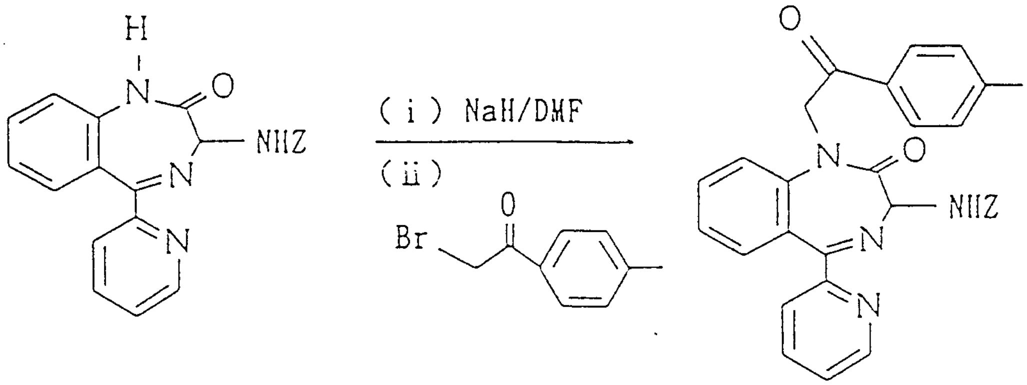


3-Benzyloxycarbonylamino-1,3-dihydro-5-(2-pyridyl)-2H-1,4-benzodiazepin-2-one hydrate (404 mg, 1 mmol) was azeotropically boiled with dimethylformamide, and to this stirred dimethylformamide (10 ml) solution was added sodium hydride (42 mg, 1.4 equivalent, 80% oil preparation). After stirring at 0 ° C for 45 minutes, the resulting mixture was treated with 2-bromo-4'-methylacetophenone (320 mg, 1.5 mmol). After stirring at room temperature for 3 hours, the reaction mixture was concentrated. The mixture was distributed between ethyl acetate and a 5% aqueous potassium bicarbonate solution, and the organic layer was filtered (Whatman IPS filter paper) and distilled off. The residue was purified by flash chromatography (eluent, 70% ethyl acetate-hexane) to obtain 3-benzyloxycarbonylamino-1,3-dihydro-1-(4'-methylphenacyl)-5-(2-pyridyl)-2H-1,4-benzodiazepin-2-one (305 mg, 59%).

NMR ($CDCl_3$)

δ: 8.65 (1H, d, J=8Hz), 8.20 (1H, d, J=8Hz), 7.90-7.35 (15H, m), 6.80 (1H, d, J=8Hz), 5.65 (1H, d, J=8Hz), 5.50 (1H, d, J=17Hz), 5.20 (1H, s), 5.10 (1H, d, J=17Hz), 2.45 (3H, s)

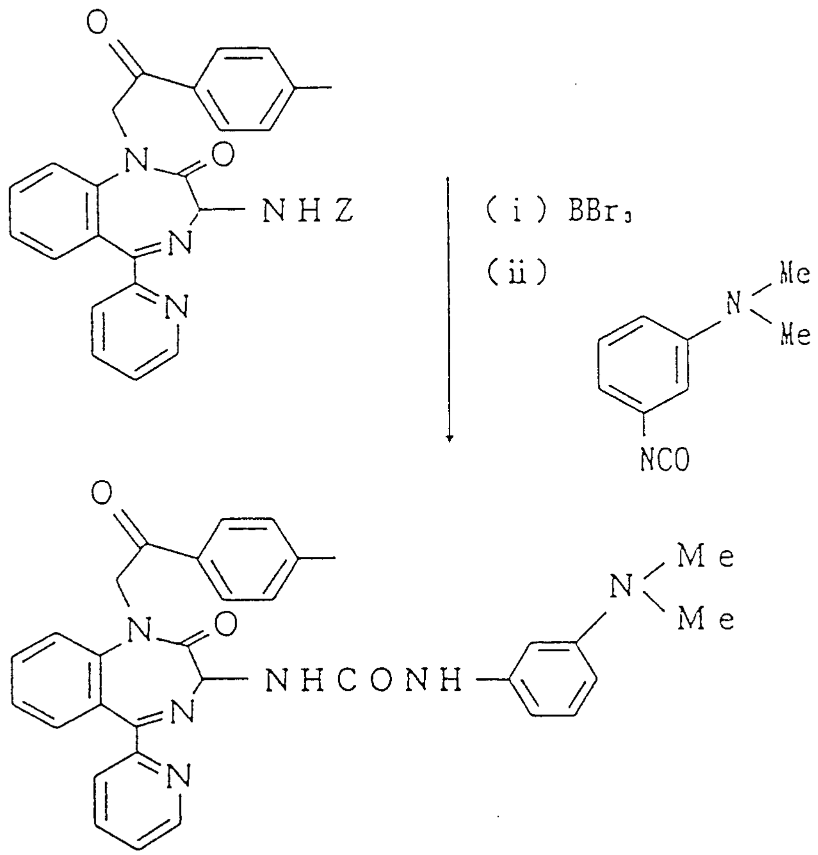


3-Benzyloxycarbonylamino-1,3-dihydro-1-(4'-methylphenacyl)-5-(2-pyridyl)-2H-1,4-benzodiazepin-2-one (305 mg, 0.59 mmol) was placed in dichloromethane at -78 °C, and treated with 1M boron tribromide (4 ml). The reaction product was warmed to room temperature and stirred for one hour. The resulting mixture was cooled to 0 °C and made basic with a cooled 1M aqueous sodium hydroxide solution. The product was extracted with chloroform, washed with an aqueous sodium chloride solution, filtered, distilled off and placed in toluene (5 ml). This was treated with a toluene solution (10 ml) of isocyanate (1 mmol). After 48 hours, the reaction product was concentrated and distributed between ethyl acetate and a 5% aqueous potassium bicarbonate solution. The organic layer was washed with an aqueous sodium chloride solution, filtered, distilled off and purified by silica gel flash chromatography (eluent, 85% ethyl acetate-hexane) to obtain 1-[2,3-dihydro-1-(4'-methylphenacyl)-2-oxo-5-(2-pyridyl)-1H-1,4-benzodiazepin-3-yl]-3-(3-dimethylaminophenyl)urea which was then freeze-dried from acetonitrile/water (78 mg, 24%, a purity of >99%).

[M+H] = 547.3

NMR ($CDCl_3$)

δ: 8.55 (1H, d, J=8Hz), 8.05 (1H, d, J=8Hz), 7.70-6.85 (16H, m), 6.45 (1H, d, J=8Hz), 6.53 (1H, d, J=8Hz), 5.75 (1H, d, J=8Hz), 5.15 (1H, d, J=18Hz), 4.95 (1H, d, J=17Hz), 2.80 (6H, s), 2.30 (3H, s)

## Claims

1. A novel benzodiazepine derivative represented by the general formula (I):

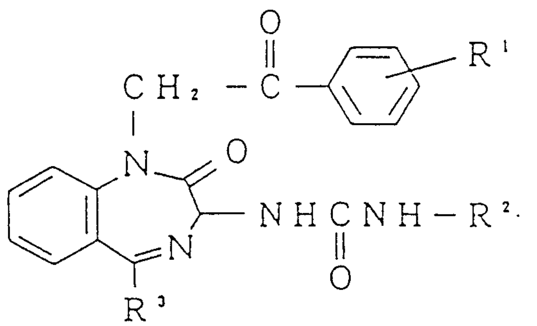

(I)

wherein $R^1$ represents a hydrogen atom, a lower alkyl group or a hydroxyl group,

$R^2$ represents (a) a phenyl group substituted with at least one of the following substituents: an amino group which may be substituted, a lower alkyl group, a cyano group, a nitro group, a carboxyl group, a lower alkoxycarbonyl group, a carbamoyl group which may be substituted or a 3- to 7-membered nitrogen-containing heterocyclic group; (b) a pyridyl group; or (c) a benzimidazolyl group; and

$R^3$ represents a phenyl group or a pyridyl group,

with the proviso that, when $R^1$ is a hydrogen atom or a lower alkyl group and $R^2$ is a lower alkyl-substituted phenyl group, then $R^3$ is a pyridyl group; or a pharmaceutically acceptable salt thereof.

2. A compound or a pharmaceutically acceptable salt thereof as claimed in claim 1, wherein $R^1$ is a lower alkyl group, $R^2$ is as defined above, except that $R^2$ is a lower alkyl-substituted phenyl group, and $R^3$ is a phenyl group.

3. A compound or a pharmaceutically acceptable salt thereof as claimed in claim 2, wherein $R^2$ is a phenyl group (which is substituted with an amino group which may be substituted, a carbamoyl group which may be substituted, or a 3- to 7-membered nitrogen-containing heterocyclic group).

4. A compound or a pharmaceutically acceptable salt thereof as claimed in claim 1, wherein $R^3$ is a pyridyl group.

5. A compound or a pharmaceutically acceptable salt thereof as claimed in claim 4, wherein $R^1$ is a lower alkyl group, and $R^2$ is a phenyl group (which is substituted with an amino group which may be substituted, or a lower alkyl group).

6. (R)-1-[2,3-Dihydro-1-(2'-methylphenacyl)-2-oxo-5-phenyl-1H-1,4-benzodiazepin-3-yl]-3-(3-dimethylaminophenyl)urea, (+)-1-[2,3-dihydro-1-(4'-methylphenacyl)-2-oxo-5-phenyl-1H-1,4-benzodiazepin-3-yl]-3-(3-dimethylaminophenyl)urea, or (±)-1-[2,3-dihydro-1-(4'-methylphenacyl)-2-oxo-5-(2-pyridyl)-1H-1,4-benzodiazepin-3-yl]-3-(3-dimethylaminophenyl]urea.

7. A process for preparing a compound represented by the formula (I):

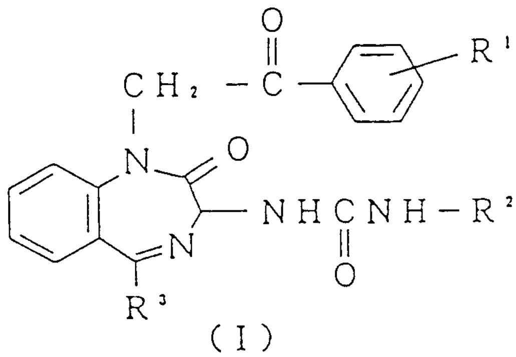

(I)

wherein $R^1$, $R^2$ and $R^3$ are as defined above, which comprises:

(a) reacting a compound represented by the formula (IIa):

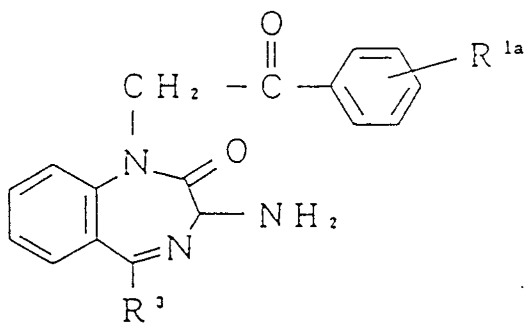

(IIa)

wherein $R^{1a}$ represents a hydrogen atom or a lower alkyl group, and $R^3$ is as defined above, with a reaction equivalent amount of a compound of the formula (III):

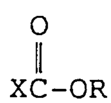

(III)

wherein R is a lower alkyl group, a phenyl group or a 4-nitrophenyl group, and X is a halogen atom) to produce a compound of the formula (IVa):

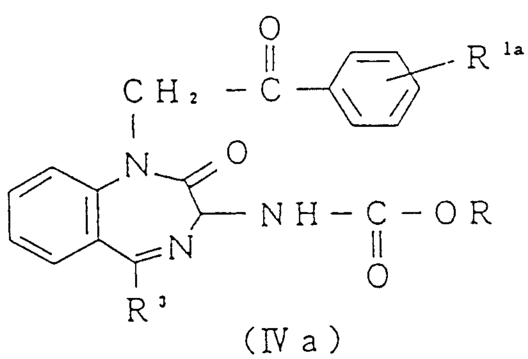

(IVa)

wherein $R^{1a}$, $R^3$ and R are as defined above, and reacting the resulting compound with a reaction equivalent amount of a compound of the formula (V):

$H_2N\text{-}R^2$ (V)

wherein $R^2$ is as defined above, to produce a compound of the formula (Ia):

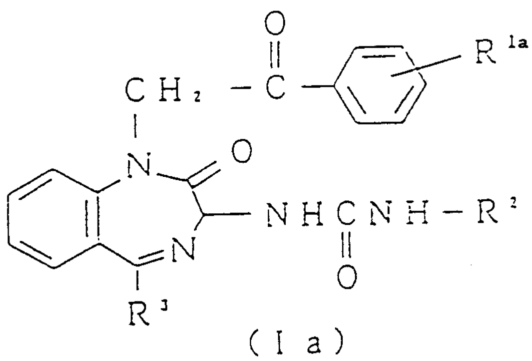

(I a)

wherein $R^{1a}$, $R^2$ and $R^3$ are as defined above; or
(b) reacting a compound of the formula (II):

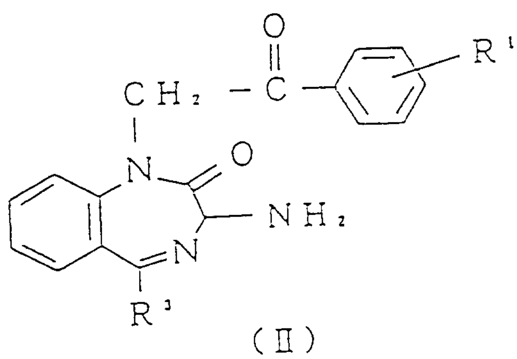

(II)

wherein $R^1$ and $R^3$ are as defined above, with a reaction equivalent amount of a compound of the formula (VI):

$OCN\text{-}R^2$ (VI)

wherein $R^2$ is as defined above, which can be commercially available or can be prepared just before use.

**8.** A pharmaceutical composition comprising a compound represented by the above-described formula (I) or a pharmaceutically acceptable salt thereof, and a pharmaceutically acceptable diluting agent and/or carrier for the pharmaceutical preparation.

**9.** A pharmaceutical composition as claimed in claim 8 having a CCK-B receptor antagonistic activity or a gastrin receptor antagonistic activity.

**10.** A pharmaceutical composition as claimed in claim 9, which is an agent for the treatment of diseases induced by physiological function disorders controlled by gastrin, in particular, gastric ulcer, duodenum ulcer, gastritis, reflux esophagitis, Zollinger-Ellison syndrome, without causing side-effects relating to the CCK-A receptor.

**11.** A pharmaceutical composition as claimed in claim 9, which is an agent for the treatment of diseases induced by physiological function disorders controlled by gastrin B receptor, in particular, appetite controlling system disorders and pain, or the central nervous system disorders such as an antianxiety

agent, without causing side-effects relating to the CCK-A receptor.

## INTERNATIONAL SEARCH REPORT

International application No.
PCT/JP93/00844

**A. CLASSIFICATION OF SUBJECT MATTER**

Int. $Cl^5$ C07D243/24, A61K31/55

According to International Patent Classification (IPC) or to both national classification and IPC

**B. FIELDS SEARCHED**

Minimum documentation searched (classification system followed by classification symbols)

Int. $Cl^5$ C07D243/06-243/38, A61K31/55

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| Y | JP, A, 61-63666 (Merck & Co., Inc.), April 1, 1986 (01. 04. 86), Line 5, lower left column, page 1 to line 16, lower left column, page 2 and line 10, upper right column to line 7, lower left column, page 21 (Family: none) | 1-11 |
| A | "NATURE" 312, page 363 (1984) | 1-11 |

☐ Further documents are listed in the continuation of Box C.  ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| September 10, 1993 (10. 09. 93) | October 5, 1993 (05. 10. 93) |
| Name and mailing address of the ISA/ Japanese Patent Office | Authorized officer |
| Facsimile No. | Telephone No. |